# EUROPEAN PATENT SPECIFICATION

(11) **EP 4 346 780 B1**
(45) Date of publication and mention of the grant of the patent: **04.11.2026**
(21) Application number: 22730207.2
(22) Date of filing: 23.05.2022
(51) Int. Cl.: A61K 9/48

(54) **HARD-SHELL CAPSULE WITH INFLUX PREVENTION OF GASTRIC FLUIDS**
HARTSCHALENKAPSEL MIT ZUFLUSSVERHINDERUNG VON MAGENFLÜSSIGKEITEN
CAPSULE À ENVELOPPE DURE AVEC PRÉVENTION D'AFFLUX DE FLUIDES GASTRIQUES

(30) Priority: 25.05.2021 EP 21175704
(43) Date of publication of application: 10.04.2024
(73) Proprietor: Evonik Operations GmbH, 45128 Essen (DE)
(72) Inventor: BÄR, Hans, 64720 Michelstadt (DE); HELLER, Philipp, 55116 Mainz (DE); SMITH, Steven, 63263 Neu-Isenburg (DE); HÖLZER, Bettina, 64625 Bensheim (DE)
(74) Representative: Evonik Patent Association
(86) International application number: PCT/EP2022/063843
(87) International publication number: WO 2022/248381

(56) References cited:
- WO-A1-2019/096833
- WO-A1-2019/148278
- WO-A1-2020/229178
- WO-A1-2020/229192
- US-A1- 2010 291 201

## Description

### Field of the invention

The invention refers to a process for preparing a polymer-coated hard shell capsule comprising at least a functional coat and a top coat, suitable as container for pharmaceutical or nutraceutical biologically active ingredients, wherein the hard shell capsule comprises a body and a cap, wherein in the closed state the cap overlaps the body either in a pre-locked state or in a final-locked state, wherein the hard shell capsule is provided in the pre-locked state and
is coated
with a first coating solution, suspension or dispersion comprising or consisting of
   a1) at least one polymer;
   b1) optionally at least one glidant;
   c1) optionally at least one emulsifier;
   d1) optionally at least one plasticizer;
   e1) optionally at least one biologically active ingredient; and
   f1) optionally at least one additive, different from a1) to e1);
to obtain the functional coat of the hard shell capsule in the pre-locked state; and thereafter is coated with a second coating solution, suspension or dispersion, which is different from the first coating solution, suspension or dispersion, comprising or consisting of
   a2) at least one polymer;
   b2) optionally at least one glidant;
   c2) at least one emulsifier;
   d2) at least one plasticizer;
   e2) optionally at least one biologically active ingredient; and
   f2) optionally at least one additive, different from a2) to e2);
to obtain the top coat of the hard shell capsule in the pre-locked state, wherein
the total coating amount is 2.0 to 10 mg/cm²; and
the coating amount of the top coat is at most 40 % of the coating amount of the functional coat. Furthermore, the invention refers to a polymer-coated hard shell capsule obtained from the process according to the invention and the use of the polymer-coated hard shell capsule for immediate, delayed or sustained release.

### Background

During the Covid crisis nucleic acid based drugs have gained a major role in fighting this global pandemic. However, the production, storage, use, and delivery of such drugs, based on liquid nanoparticles (LNPs) is challenging, *inter alia* due to the temperature sensitivity of the nucleic acids and their tendency to lose their activity through degradation when in contact with different media. At present, the drugs in the market are predominantly administered via injection of solutions. In order to provide an oral delivery route for such nucleic acid based drugs, a vehicle is therefore required which allows temperature sensitive handling, including filing of the nucleic acid based drugs, as well as the prerequisite that the GI tract can be passed without enzymatically degrading the nucleic acid. Furthermore, due to the demand of extremely high amounts of those nucleic acid based drugs, the production methods needs to be efficient and fast.

For example, Ball et al., Oral delivery of siRNA lipid nanoparticles: Fate in the GI tract, Scientific reports (2018) 8:2178 discloses the behaviour of LNPs in gastric fluid.

In particular, it is disclosed that first LNPs were incubated in simulated gastric fluid that contained pepsin (pH 1-2). After 30 minutes, they were exposed to a simulated intestinal fluid containing pancreatin and incubated for additional 30 minutes. Gene silencing was assessed 24 hours later by quantitative PCR. The digested LNPs were ineffective, while the undigested LNPs achieved ~70% gene silencing. Potency may have been reduced by aggregation of the LNPs, as seen in the significant increase in the z-average diameter and PDI of the digested LNPs compared to the original nanoparticles. It is disclosed that degradation effects caused by intestinal fluids containing pancreatin (mixture of enzymes that include trypsin, amylase, lipase, ribonuclease, and proteases) can only be resolved by modifications of the nanoparticle formulation or with other excipients which become part of the capsule filling.

WO 2019/096833 A1 discloses a process for preparing a polymer coated hard shell capsule. The process comprises first coating a solution comprising a polymer to obtain a polymer first coated hard shell capsule in pre-locked state. The polymer coated hard shell capsule in pre-locked capsule is further coated with a topcoat solution having a polymer and optionally glidants.

WO 2020/229178 A1 discloses a process for preparing a polymer coated hard shell capsule. The process comprises first coating with a solution comprising a polymer to obtain a polymer coated hard shell capsule in pre-locked state. The polymer coated hard shell capsule in pre-locked state is further coated with a topcoat solution comprising a polymer. Examples 6 and 7 of D2 discloses a process comprising first coating a solution comprising a polymer to obtain a polymer coated hard shell capsule in pre-locked state. The polymer coated hard shell capsule in pre-locked state is further coated with a topcoat solution consisting of HPMC polymer.

WO 2020/229192 A1 discloses a process for preparing a polymer coated hard shell capsule. The process comprises coating with a solution comprising a polymer to obtain a polymer coated hard shell capsule in pre-locked state.

WO 2019/148278 A1 discloses a process for preparing a polymer coated hard shell capsule in a final locked state. The process comprises first coating with a solution comprising a polymer to obtain a polymer seal coated hard shell capsule which is further coated with a HPMCAS polymer to obtain the top coated capsule.

US 2010/291201 A1 discloses a capsule coated with a drug layer coat, followed by a seal coating over the drug layer coating. The capsule may further comprise a coating of immediate release coatings, protective coatings, enteric or delayed release coatings, sustained release coatings, barrier coatings, and combinations thereof.

Another option for oral delivery could be the use of hard shell capsules. Hard shell capsules, which are filled with the LNPs and locked and thereafter coated, as for example disclosed in WO 2019/148278 A1 and US 2010 291201 A1, are not suitable, since the coating process can lead to degradation of the nucleic acids due to their temperature sensitivity.

In order to overcome these issues, the inventors of the present invention started by employing polymer-coated hard shell capsules as for example disclosed in WO 2019/096833 A1, which are coated in pre-locked state and later filled. However, in the field of oral delivery of polymer-coated hard shell capsules the main target was to provide a hard shell capsule, which prevents release of the drug in gastric fluid. In this regard, it was found that coatings which prevent release when incubated in simulated gastric fluid not necessarily prevent influx of gastric fluid into the capsule shell. Even though a release of the drug is not observed in known those hard shell capsules, the influx of gastric fluid foster pepsin mediated digestion even before release of the nucleic acid based drug substance from the capsule.

Therefore, the inventors of the present invention developed an optimized modified release (enteric) pre-coated empty hard-shell capsule which can limit the influx of the simulated gastric fluid during an incubation period of two hours to a level below 5% loss on drying increase of the capsule filling and are suitable to be successfully employed in capsule filling machines. Particularly desirable are capsules which have a low average media uptake, preferably below 3 %, in order to avoid detrimental effects on sensitive pharmaceutical or nutraceutical biologically active ingredients like nucleic acids.

In order to obtain such hard-shell capsules, it has been found that a functional coat and a top coat are mandatory, as well as a specific coating amount of 2.0 to 10 mg/cm² and that the coating amount of the top coat is at most 40%, at most 30 %, preferably at most 28% of the coating amount of the functional coat.

### Summary of the invention

In a first aspect the invention refers to a process for preparing a polymer-coated hard shell capsule comprising at least a functional coat and a top coat, suitable as container for pharmaceutical or nutraceutical biologically active ingredients, wherein the hard shell capsule comprises a body and a cap, wherein in the closed state the cap overlaps the body either in a pre-locked state or in a final-locked state, wherein the hard shell capsule is provided in the pre-locked state and
is coated
with a first coating solution, suspension or dispersion comprising or consisting of
   a1) at least one polymer;
   b1) optionally at least one glidant;
   c1) optionally at least one emulsifier;
   d1) optionally at least one plasticizer;
   e1) optionally at least one biologically active ingredient; and
   f1) optionally at least one additive, different from a1) to e1);
to obtain the functional coat of the hard shell capsule in the pre-locked state; and thereafter is coated with a second coating solution, suspension or dispersion, which is different from the first coating solution, suspension or dispersion, comprising or consisting of
   a2) at least one polymer;
   b2) optionally at least one glidant;
   c2) at least one emulsifier;
   d2) at least one plasticizer;
   e2) optionally at least one biologically active ingredient; and
   f2) optionally at least one additive, different from a2) to e2);
to obtain the top coat of the hard shell capsule in the pre-locked state, wherein
the total coating amount is 2.0 to 10 mg/cm²; and
the coating amount of the top coat is at most 40 % of the coating amount of the functional coat.

In a second aspect the invention refers to a polymer-coated hard shell capsule obtained from the process according to the present invention.

In a third aspect the invention refers to the use of the polymer-coated hard shell capsule according to the present invention for immediate, delayed or sustained release.

### Detailed description of the invention

### Hard shell capsules

Hard shell capsules for pharmaceutical or nutraceutical purposes are well known to a skilled person. A hard shell capsule is a two-piece encapsulation capsule comprising of the two capsule halves, called the body and the cap. The capsule body and cap material is usually made from a hard and sometimes brittle material. The hard shell capsule comprises a body and a cap. Body and cap are usually of a one end open cylindrical form with closed rounded hemispherical ends on the opposite end. The shape and size of the cap and body are such that the body can be pushed telescopically with its open end into the open end of the cap.

The body and the cap comprise a potential overlapping, matching area (overlap area) outside the body and inside the cap which partially overlap when the capsule is closed in the pre-locked state and totally overlap in the final-locked state. When the cap is partially slid over the overlapping matching area of the body the capsule is in the pre-locked state. When the cap is totally slid over the overlapping matching area of the body the capsule is in the final-locked state. The maintenance of the pre-locked state or of the final-locked state is usually supported by snap-in locking mechanisms of the body and the cap such as matching encircling notches or dimples, preferably elongated dimples.

Usually the body is longer than the cap. The outside overlapping area of the body can be covered by the cap in order to close or to lock the capsule. In the closed state the cap covers the outside overlap area of the body either in a pre-locked state or in a final-locked state. In the final-locked state the cap covers the outside overlap area of the body in total, in the pre-locked state the cap overlaps the outside overlapping area of the body only partially. The cap can be slid over the body to be fixed in usually one of two different positions in which the capsule is closed either in a pre-locked state or in a final-locked state.

Hard shell capsules are commercially available in different sizes. Hard shell capsules are usually delivered as empty containers with the body and cap already positioned in the pre-locked state and on demand as separate capsules halves, bodies and caps. The pre-locked hard shell capsules can be provided to a capsule-filling machine, which performs the opening, filling and closing of the capsule into the final-locked state. Usually hard shell capsules are filled with dry materials, for instance with powders or granules, or viscous liquids comprising a biologically active ingredient.

The cap and body are provided with closure means that are advantageous for the pre-locking (temporary) and/or final locking of the capsule. Therefore, elevated points can be provided on the inner wall of the cap and somewhat larger indented points are provided on the outer wall of the body, which are arranged so that when the capsule is closed the elevations fit into the indentations. Alternatively, the elevations can be formed on the outer wall of the body and the indentations on the inner wall of the cap. Arrangements in which the elevations or indentations arranged in a ring or spiral around the wall. Instead of the point-like configuration of the elevations and indentations, these may encircle the wall of the cap or body in an annular configuration, although advantageously recesses and openings are provided which enable an exchange of gases into and out of the capsule interior. One or more elevations can be provided in an annular arrangement around the inner wall of the cap and the outer wall of the body such that, in the final-locked position of the capsule, an elevation on the cap is located adjacent to an elevation on the body. Sometimes elevations are formed on the outside of the body close to the open end and indentations are formed in the cap close to the open end such that the elevations on the body latch into the indentations in the cap in the final-locked position of the capsule. The elevations can be such that the cap can be opened in the pre-locked state at any time without damage to the capsule or, alternatively, so that once it has been closed the capsule cannot be opened again without destroying it. Capsules with one or more such latching mechanisms (latches) (for example two encircling grooves) are preferred. More preferred are capsules with at least two such latching means which secure the two capsule parts to different degrees. In a part of this kind, a first latching (dimples or encircling notches) means can be formed close to the openings in the capsule cap and the capsule body and a second latching (encircling notches) can be shifted somewhat further towards the closed end of the capsule parts. The first latching means secure the two capsule parts less strongly than the second does. This variant has the advantage that after the production of the empty capsules the capsule cap and capsule body can initially be pre-locked joined together using the first latching mechanism. In order to fill the capsule the two capsule parts are then separated again. After filling, the two capsule parts are pushed together until the second set of latches firmly secures the capsule parts in a final-locked state.

Preferably, the body and the cap of the hard shell capsule are comprising each encircling notches and/or dimples in the area, where the cap can be slid over the body. Encircling notches of the body and dimples of the cap match to each other to provide a snap-in or snap into-place mechanism. The dimples can be circular or elongated (oval) in the longitudinal direction. Encircling notches of the body and encircling notches of the cap (closely matched rings) also match to each other to provide a snap-in or snap into-place mechanism. This allows the capsule to be closed by a snap-into-place mechanism either in a pre-locked state or in a final-locked state.

Preferably, matching encircling notches of the body and elongated dimples of the cap are used to fix the body and the cap to each other in the pre-locked state. Matching encircling notches of the body and the cap are preferably used to fix or lock the body and the cap to each other in the final-locked state.

The area, where the cap can be slid over the body can be called the overlapping area of the body and the cap or briefly the overlap area. If the cap overlaps the body only partially, maybe to 20 to 90 or 60 to 85 % of the overlap area, the hard shell capsule is only partially closed (pre-locked). Preferably, in the presence of a locking mechanism, like matching encircling notches and/or dimples in body and cap, the partially closed capsule can be called pre-locked. When the capsule is polymer-coated in the pre-locked state the coating will cover the completely outer surface including that part of the overlap area of the body and cap that is not overlapped by the cap in this pre-locked state. When the capsule is polymer-coated in the pre-locked state and then closed to the final-locked state the coating of that part of the overlap area of the body and cap that was not overlapped by the cap in the pre-locked state will then become covered by the cap. The presence of that part of the coating which is then enclosed in the final-locked state between the body and the cap is sufficient for the hard shell capsule to be tightly sealed.

If the cap overlaps the body the total overlapping area of the body, the hard shell capsule is finally closed or in the final-locked state. Preferably, in the presence of a locking mechanism, like matching encircling notches and/or dimples in body and cap, the finally closed capsule can be called final-locked.

Usually dimples are preferred for the fixing the body and the cap in the pre-locked state. As a non-binding rule the matching area of dimples is smaller than the matching area of encircling notches. Thus snapped-in dimples can be snapped-out again by applying less forces than those that would be necessary to snap-out a snapped-in fixation by matching encircling notches.

The dimples of the body and cap are located in the area, where the cap can be slid over the body match to each other in the pre-locked state by a snap in or snap into-place mechanism. There can be for example 2, 4, or preferably 6 notches or dimples located distributed circular around the cap.

Usually the dimples of the cap are and the encircling notches of the body in the area, where the cap can be slid over the body match to each other so that they that allow the capsule to be closed by a snap-into-place mechanism in the pre-locked state. In the pre-locked state, the hard shell capsule can be re-opened manually or by a machine without damaging, because the forces needed to open are comparatively low. Thus, the "pre-locked state" is sometimes designated also as "loosely capped".

Usually the encircling notches or matching locking rings of the body and the cap in the area, where the cap can be slid over the body match to each other so that they that allow the capsule to be closed by a snap-into-place mechanism in the final-locked state. In the final-locked state, the hard shell capsule cannot or can be only hardly be re-opened manually or by a machine without damaging, because the forces needed to open are comparatively high.

Usually dimples and the encircling notches are formed in the capsule body or capsule cap. When the capsule parts provided with these elevations and indentations are fitted into one another, ideally defined uniform gaps of from 10 microns to 150 microns, more particularly 20 microns to 100 microns, are formed along the contact surface between the capsule body and the capsule cap placed thereon.

Preferably, the body of the hard shell capsule comprises a tapered rim. The tapered rim prevent the rims of the body and the cap to collide and becoming damaged when the capsule is closed manually or by a machine.

In contrast to a hard shell capsule, a soft shell capsule is a welded one piece encapsulation capsule. A soft gel capsule is often made from blow molded soft gelling substances and is usually filled with liquids comprising a biologically active ingredient by injection. The invention is not concerned with welded soft shell one piece encapsulation capsules.

### Sizes of hard shell capsules

A closed, final-locked hard shell capsule can have a total length in the range from about 5 to 40 mm. The diameter of the cap can be in the range from about 1.3 to 12 mm. The diameter of the body can be in the range from about 1.2 to 11 mm. The length of the cap can be in the range from about 4 to 20 mm and that of the body in the range from 8 to 30 mm. The fill volume can be between about from 0.004 to 2 ml. The difference between the pre-locked length and the final-locked length can be about 1 to 5 mm.

Capsules can be divided into standardized sizes for example from sizes 000 to 5. A closed capsule of size 000 has, for example, a total length of about 28 mm with an outer diameter of the cap of about 9.9 mm and an outer diameter of the body of about 9.5 mm. The length of the cap is about 14 mm, that of the body about 22 mm. The fill volume is about 1.4 ml.

A closed capsule of size 5 has, for example, a total length of about 10 mm and an outer diameter of the cap of about 4.8 mm and an outer diameter of the body of about 4.6 mm. The length of the cap is about 5.6 mm, that of the body about 9.4 mm. The fill volume is about 0.13 ml.

A size 0 capsule may show a length of about 23 to 24 mm in the pre-locked state and of about 20.5 to 21.5 mm in the final-locked state. Thus, the difference between the pre-locked length and the final-locked length can be about 2 to 3 mm.

### Coated hard shell capsule

The invention is concerned with a polymer-coated hard shell capsule, obtained by the process as described herein.

### Material of the body and the cap

The base material of the body and the cap can be selected from hydroxypropyl methyl cellulose, starch, gelatin, pullulan and a copolymer of C1- to C4-alkylester of (meth)acrylic acid and (meth)acrylic acid. Preferred are hard shell capsules where body and cap are comprising or consisting of HPMC or gelatin, most preferred is HPMC because of its good adhesion properties for the polymer coating.

### Polymer or polymer mixture comprised in the functional or top coating layer

In the following polymers, which are suitable for being used at the at least one polymer in the functional or top coating layer are disclosed. If not specifically described otherwise, the respective polymer can in general be used in both coating layers (in the following referred to as "coating layer").

The at least one polymer comprised in the coating layer is preferably a film-forming polymer and can be selected from the group of anionic polymers, cationic polymers and neutral polymers or any mixture thereof.

The selection of generic or specific polymer features or embodiments as disclosed herein can be combined without restriction with any other generic or specific selection of material or numerical features or embodiments as disclosed herein, such as capsule materials, capsule sizes, coating thicknesses, biologically active ingredients and any other features or embodiments as disclosed.

The coating layer, which can be a single layer or can comprise or consist of two or more individual layers, can comprise in total 10 to 100, 20 to 95, 30 to 90 % by weight of one or more polymers, preferably (meth)acrylate copolymer(s).

The proportions of monomers mentioned for the respective polymers in general add up to 100% by weight.

The functional coating layer and the top coating layer are different from each other. In particular, they differ in at least one polymer, which is respectively contained in the coating layer. For example, both layers can contain the same anionic polymer, however it is then necessary that one of the two coatings contains a second polymer different from the specific anionic polymer.

In a preferred embodiment the functional coating layer comprises at least one anionic polymer and/or comprises at least one polymer having a T_{gm} of less than 50 °C. In a more preferred embodiment, the functional coating layer comprises at least one anionic (meth)acrylate copolymer, preferably as described below.

In a preferred embodiment the top coating layer comprises at least one cationic polymer or at least one neutral polymer or any mixture thereof. In a preferred embodiment the top coating layer is selected from at least one natural polymer or a starch, preferably as described below.

### Glass transition temperature T_{gm}

The coating layer can comprise one or more polymers, preferably (meth)acrylate copolymer(s), with a glass transition temperature *T_{gm}* of 125 °C or less, preferably from -10 to 115 °C.

The coating layer can comprise one or more anionic cellulose(s), ethyl cellulose and/or one or more starches comprising at least 35 % by weight amylose with a glass transition temperature *T_{gm}* of 130 or less, preferably 127 °C or less, more preferably from 50 to 127 °C.

The glass transition temperature *T_{gm}* according to the present invention is preferably determined by Differential Scanning Calorimetry (DSC) according to ISO 11357-2:2013-05. The determination is performed with a heating rate of 20 K/min. The glass transition temperature *T_{gm}* can as well be determined by half step height method as described in section 10.1.2 of DIN EN ISO 11357-2.

### Anionic polymers - enteric coating and gastric resistance

The described process is especially useful for providing tightly closed polymer-coated hard shell capsules for pharmaceutical or nutraceutical dosage forms with gastric resistance and an intended rapid release in the small intestine (enteric coating) or large intestine (colon targeting).

The at least one polymer comprised in the coating layer can be an anionic polymer selected from the groups of anionic (meth)acrylate copolymers, anionic polyvinyl polymers or copolymers and anionic celluloses.

The above-mentioned anionic polymers are also called "enteric polymers". In the coating layer such polymers are capable of providing enteric protection to the capsule. Enteric protection shall mean, when the capsule is in the final closed state and comprises a fill comprising a pharmaceutical or nutraceutical biologically active ingredient, less than 10 % of the comprised biologically active ingredient will be released after 120 min in 0.1 HCl, pH 1.2. Most preferred after 120 min in 0.1 HCl pH 1.2 and subsequent change to a buffered medium of pH 6.8 about 80 % or more of the comprised biologically active ingredient will be released after a total time of 165 min or 180 min. Colon targeting shall mean, when the capsule is in the final closed state and comprises a fill comprising a pharmaceutical or nutraceutical biologically active ingredient, less than 10 % of the comprised biologically active ingredient will be released after 120 min in 0.1 HCl, pH 1.2. Preferred after 120 min in 0.1 HCl pH 1.2 and subsequent change to a buffered medium of pH 6.8 about 80 % or more of the comprised biologically active ingredient will be released after a total time of 165 min. Most preferred after 120 min in 0.1 HCl pH 1.2 and 60 min at a subsequent intermediate change to a buffered medium of pH 6.5 or 6.8 and subsequent final change to a buffered medium of pH 7.2 or pH 7.4 about 80 % or more of the comprised biologically active ingredient will be released after a total time of 225 min or 240 min.

The dissolution test is performed according to the United States Pharmacopeia 43 (USP) chapter <711> utilizing USP Apparatus II with a paddle speed of 50 or 75 rpm. The test media temperature will be adjusted to 37 + 0.5°C. Samples will be taken at appropriate time points.

### Anionic (meth)acrylate copolymers

Preferably the anionic (meth)acrylate copolymer comprises 25 to 95, preferably 40 to 95, in particular 60 to 40, % by weight free-radical polymerized C1- to C12-alkyl esters, preferably C1- to C4-alkyl esters of acrylic or of methacrylic acid and 75 to 5, preferably 60 to 5, in particular 40 to 60 % by weight (meth)acrylate monomers having an anionic group. The proportions mentioned in general add up to 100% by weight. However, it is also possible in addition, without this leading to an impairment or alteration of the essential properties, for small amounts in the region of 0 to 10, for example 1 to 5, % by weight of further monomers capable of vinylic copolymerization, such as, for example, hydroxyethyl methacrylate or hydroxy-ethyl acrylate, to be present. It is preferred that no further monomers capable of vinylic copolymerization are present.

C1- to C4-alkyl esters of acrylic or methacrylic acid are in particular methyl methacrylate, ethyl methacrylate, butyl methacrylate, methyl acrylate, ethyl acrylate and butyl acrylate.

A (meth)acrylate monomer having an anionic group is, for example, acrylic acid, with preference for methacrylic acid.

Suitable anionic (meth)acrylate copolymers are those polymerized from of 40 to 60% by weight methacrylic acid and 60 to 40% by weight methyl methacrylate or 60 to 40% by weight ethyl acrylate (EUDRAGIT^{®} L or EUDRAGIT^{®} L 100 55 types).

EUDRAGIT^{®} L is a copolymer polymerized from 50% by weight methyl methacrylate and 50% by weight methacrylic acid. The pH of the start of the specific active ingredient release in intestinal juice or simulated intestinal fluid can be stated to be at about pH value 6.0.

EUDRAGIT^{®} L 100-55 is a copolymer polymerized from 50% by weight ethyl acrylate and 50% by weight methacrylic acid. EUDRAGIT^{®} L 30 D-55 is a dispersion comprising 30% by weight EUDRAGIT^{®} L 100-55. The pH of the start of the specific active ingredient release in intestinal juice or simulated intestinal fluid can be stated to be at about pH value 5.5.

Likewise, suitable are anionic (meth)acrylate copolymers polymerized from 20 to 40% by weight methacrylic acid and 80 to 60% by weight methyl methacrylate (EUDRAGIT^{®} S type). The pH value of the start of the specific active ingredient release in intestinal juice or simulated intestinal fluid can be stated to be at about pH value 7.0.

Suitable (meth)acrylate copolymers are polymerized from 10 to 30% by weight methyl methacrylate, 50 to 70% by weight methyl acrylate and 5 to 15% by weight methacrylic acid (EUDRAGIT^{®} FS type). The pH at the start of the specific active ingredient release in intestinal juice or simulated intestinal fluid can be stated to be at about pH value 7.0.

EUDRAGIT^{®} FS is a copolymer polymerized from 25% by weight methyl methacrylate, 65% by weight methyl acrylate and 10% by weight methacrylic acid. EUDRAGIT^{®} FS 30 D is a dispersion comprising 30% by weight EUDRAGIT^{®} FS.

Suitable is a copolymer composed of
20 to 34% by weight methacrylic acid and/or acrylic acid,
20 to 69% by weight methyl acrylate and
0 to 40% by weight ethyl acrylate and/or where appropriate
0 to 10% by weight further monomers capable of vinylic copolymerization,
with the proviso that the glass transition temperature of the copolymer according to ISO 11357-2:2013-05, subsection 3.3.3, is not more than 60°C. This (meth)acrylate copolymer is particularly suitable, because of its good elongation at break properties, for compressing pellets to tablets.

Suitable is a copolymer polymerized from
20 to 33% by weight methacrylic acid and/or acrylic acid,
5 to 30% by weight methyl acrylate and
20 to 40% by weight ethyl acrylate and
more than 10 to 30% by weight butyl methacrylate and where appropriate
0 to 10% by weight further monomers capable of vinylic copolymerization,
where the proportions of the monomers add up to 100% by weight,
with the proviso that the glass transition temperature of the copolymer according to ISO 11357-2:2013-05, subsection 3.3.3 (midpoint temperature Tmg), is 55 to 70°C.

The copolymer preferably consists of 90, 95 or 99 to 100% by weight of the monomers methacrylic acid, methyl acrylate, ethyl acrylate and butyl methacrylate in the ranges of amounts indicated above. However, it is possible, without this necessarily leading to an impairment of the essential properties, for small amounts in the range from 0 to 10, e.g. 1 to 5% by weight of further monomers capable of vinylic copolymerization additionally to be present, such as, for example, methyl methacrylate, butyl acrylate, hydroxyethyl methacrylate, vinylpyrrolidone, vinyl-malonic acid, styrene, vinyl alcohol, vinyl acetate and/or derivatives thereof.

Further suitable anionic (meth)acrylate copolymers can be so called core/shell polymers as described in WO 2012/171575 A2 or WO 2012/171576 A1. A suitable Core Shell polymer is a copolymer from a two stage emulsion polymerization process with a core of 75 % by weight comprising polymerized units of 30% by weight of ethyl acrylate and 70% by weight of methyl methacrylate and a shell of polymerized units comprising 25 % by weight of polymerized from 50% by weight ethyl acrylate and 50% by weight methacrylic acid.

A suitable Core-Shell polymer can be a copolymer from a two stage emulsion polymerization process with a core with 70 to 80 % by weight, comprising polymerized units of 65 to 75 % by weight of ethyl acrylate and 25 to 35 % by weight of methyl methacrylate, and a shell with 20 to 30 % by weight, comprising polymerized units of 45 to 55 % by weight ethyl acrylate and 45 to 55 % by weight methacrylic acid.

### Anionic celluloses

Anionic celluloses can be selected from carboxymethyl ethyl cellulose and its salts, cellulose acetate phthalate (CAP), cellulose acetate succinate (CAS), cellulose acetate trimellitate (CAT), hydroxypropyl methyl cellulose phthalate (HPMCP, HP50, HP55), hydroxypropyl methyl cellulose acetate succinate (HPMCAS-LF, -MF, -HF).

The coating layer can comprise one or more anionic cellulose(s), ethyl cellulose and/or one or more starches comprising at least 35 % by weight amylose, preferably with a glass transition temperature *T_{gm}* of 130 °C or less (determined by Differential Scanning Calorimetry (DSC) according to ISO 11357-2:2013-05), wherein the coating layer is preferably present in an amount of about 1 to 5.8, more preferably 2 to 5 mg/cm².

The coating layer can comprise in total 10 to 100, 20 to 95, 30 to 90 % by weight of one or more anionic cellulose(s), ethyl cellulose and/or one or more starches comprising at least 35 % by weight amylose.

The glass transition temperature *T_{gm}* of hydroxypropyl methyl cellulose phthalate is about 132 to 138 °C (type HP-55 about 133 °C, type HP-50 about 137 °C).

The glass transition temperature *T_{gm} of* hydroxypropyl methyl cellulose acetate succinate (HPMCAS) is about 120 °C (AquaSolve^{™} L HPMCAS 119°C, AquaSolve^{™} M HPMCAS 120°C, AquaSolve^{™} H HPMCAS 122°C).

### Ethyl cellulose

Ethyl cellulose is a derivative of cellulose in which some of the hydroxyl groups of the repeating glucose units are converted into ethyl ether groups. Ethyl cellulose can be used as a delayed release coating material for the capsules as disclosed. The glass transition temperature *T_{gm}* of ethyl cellulose can be in the range of about 128 to 130 °C (Hui Ling Lai et al. Int.J.Pharmaceuticals 386 (2010) 178-184).

### Starches comprising at least 35 % by weight amylose

Starches comprising at least 35 % by weight amylose are commercially available as starch from corn or maize origin.

Starches comprising at least 35 % by weight amylose are known for example from EP 1296658 B1. This type of chemically modified (acetylated) starch with a high content in amylose is obtained through a pre-gelation process. These starches show a high mechanical resistance for the production of capsules and coatings for solid formulations used in various application in the fields of pharmaceuticals or nutraceuticals.

The glass transition temperature *T_{gm}* of starches comprising at least 35 % by weight amylose can be in the range of about 52 to 60 °C (Peng Liu et al., J.Cereal Science (2010) 388-391).

### Anionic vinyl copolymers

Anionic vinyl copolymers can be selected from unsaturated carboxylic acids other than acrylic acid or methacrylic acid as exemplified by polyvinylacetatephthalate or a copolymer of vinylacetate and crotonic acid (preferably at a ratio of 9:1).

### Cationic polymers

A suitable cationic (meth)acrylate copolymer comprised in the coating layer can be polymerized from monomers comprising C1- to C4-alkyl esters of acrylic or of methacrylic acid and an alkyl ester of acrylic or of methacrylic acid with a tertiary or a quaternary ammonium group in the alkyl group. The cationic, water-soluble (meth)acrylate copolymer can be polymerized partly or fully of alkyl from acrylates and/or alkyl methacrylates having a tertiary amino group in the alkyl radical. A coating comprising these kind of polymers may have the advantage of providing moisture protection to the hard shell capsule. Moisture protection shall be understood a reduced uptake of moisture or water during storage of the readily filled and final-locked capsules.

A suitable cationic (meth)acrylate copolymer can be polymerized from 30 to 80% by weight of C1-to C4-alkyl esters of acrylic or of methacrylic acid, and 70 to 20% by weight of alkyl(meth)acrylate monomers having a tertiary amino group in the alkyl radical.

The preferred cationic (meth)acrylate copolymer can be polymerized from 20 - 30% by weight of methyl methacrylate, 20 - 30% by weight of butyl methacrylate and 60 - 40% by weight of dimethylaminoethyl methacrylate (EUDRAGIT^{®} E type polymer).

A specifically suitable commercial (meth)acrylate copolymer with tertiary amino groups is polymerized from 25% by weight of methyl methacrylate, 25% by weight of butyl methacrylate and 50% by weight of dimethylaminoethyl methacrylate (EUDRAGIT^{®} E 100 or EUDRAGIT^{®} E PO (powder form)). EUDRAGIT^{®} E 100 and EUDRAGIT^{®} E PO are water-soluble below approx. pH value 5.0 and are thus also gastric juice-soluble.

A suitable (meth)acrylate copolymer can be composed of 85 to 98% by weight of free-radical polymerized C1 to C4 alkyl esters of acrylic or methacrylic acid and 15 to 2% by weight of (meth)acrylate monomers with a quaternary amino group in the alkyl radical.

Preferred C1 to C4 alkyl esters of acrylic or methacrylic acid are methyl acrylate, ethyl acrylate, butyl acrylate, butyl methacrylate and methyl methacrylate.

Further suitable cationic (meth)acrylate polymers may contain polymerized monomer units of 2-trimethylammonium-ethyl methacrylate chloride or trimethylammonium-propyl methacrylate chloride.

An appropriate copolymer can be polymerized from 50 to 70% by weight of methyl methacrylate, 20 to 40% by weight of ethyl acrylate and 7 to 2% by weight of 2-trimethylammoniumethyl methacrylate chloride.

A specifically suitable copolymer is polymerized from 65% by weight of methyl methacrylate, 30% by weight of ethyl acrylate and 5% by weight of 2-trimethylammoniumethyl methacrylate chloride (EUDRAGIT^{®} RS).

A further suitable (meth)acrylate copolymer can be polymerized from 85 to less than 93% by weight of C1 to C4 alkyl esters of acrylic or methacrylic acid and more than 7 to 15% by weight of (meth)acrylate monomers with a quaternary amino group in the alkyl radical. Such (meth)acrylate monomers are commercially available and have long been used for release-slowing coatings.

A specifically suitable copolymer is polymerized from 60% by weight of methyl methacrylate, 30% by weight of ethyl acrylate and 10% by weight of 2-trimethylammoniumethyl methacrylate chloride (EUDRAGIT^{®} RL).

### Neutral polymers

Neutral polymers are defined as polymers which are polymerized from neutral monomers and less than 5, preferably less than 2 % by weight or most preferred no monomers with ionic groups.

Suitable neutral polymers for the coating of the hard shell capsule are methacrylate copolymers, preferably copolymers of ethyl acrylate and methyl methacrylate like EUDRAGIT^{®} NE or EUDRAGIT^{®} NM, neutral celluloses, such as methyl-, ethyl- or propyl ethers of cellulose, for instance hydroxypropyl cellulose, polyvinyl pyrrolidone, polyvinyl acetate or polyvinyl alcohol.

Neutral methacrylate copolymers are often useful in mixture with anionic (meth)acrylate copolymers.

Neutral methacrylate copolymers are polymerized from at least to an extent of more than 95% by weight, in particular to an extent of at least 98% by weight, preferably to an extent of at least 99% by weight, in particular to an extent of at least 99% by weight, more preferably to an extent of 100% by weight, of (meth)acrylate monomers with neutral radicals, especially C1- to C4-alkyl radicals.

Suitable (meth)acrylate monomers with neutral radicals are, for example, methyl methacrylate, ethyl methacrylate, butyl methacrylate, methyl acrylate, ethyl acrylate, butyl acrylate. Preference is given to methyl methacrylate, ethyl acrylate and methyl acrylate.

Methacrylate monomers with anionic radicals, for example acrylic acid and/or methacrylic acid, can be present in small amounts of less than 5% by weight, preferably not more than 2% by weight, more preferably not more than 1 or 0.05 to 1% by weight.

Suitable examples are neutral or virtually neutral (meth)acrylate copolymers polymerized from 20 to 40% by weight of ethyl acrylate, 60 to 80% by weight of methyl methacrylate and 0 to less than 5% by weight, preferably 0 to 2 or 0.05 to 1% by weight of methacrylic acid or acrylic acid.

Suitable examples are neutral or virtually neutral (meth)acrylate copolymers polymerized from 20 to 40% methyl methacrylate by weight of, 60 to 80% by weight of ethyl acrylate and 0 to less than 5% by weight, preferably 0 to 2 or 0.05 to 1% by weight of methacrylic acid or acrylic acid. (EUDRAGIT^{®} NE or EUDRAGIT^{®} NM type).

EUDRAGIT^{®} NE and EUDRAGIT^{®} NM are copolymers comprising free-radically polymerized units of 28 to 32% by weight of methyl methacrylate and 68 to 72% by weight of ethyl acrylate.

Preference is given to neutral or essentially neutral methyl acrylate copolymers which, according to WO 01/68767 A1, have been prepared as dispersions using 1 - 10% by weight of a non-ionic emulsifier having an HLB value of 15.2 to 17.3. The latter offer the advantage that there is no phase separation with formation of crystal structures by the emulsifier (EUDRAGIT^{®} NM type).

According to EP 1 571 164 A2, corresponding, virtually neutral (meth)acrylate copolymers with small proportions of 0.05 to 1% by weight of monoolefinically unsaturated C3-C8-carboxylic acids can, however, also be prepared by emulsion polymerization in the presence of comparatively small amounts of anionic emulsifiers, for example 0.001 to 1% by weight.

### Natural polymers

Especially for nutraceutical dosage forms so called "natural polymer" coatings are preferred by many customers. Natural polymers are based on a source from nature, plants, microorganisms or animals, but sometimes further chemically processed. Natural polymers for coatings can be selected from polymers such as starch, alginates or salts of alginates, preferably sodium alginate, pectin, shellac, zein, carboxymethyl-zein, modified starch, for instance EUDRAGUARD^{®} Natural, marine sponge collagen, chitosan, gellan gum. Suitable polymer mixtures may comprise:
Ethyl cellulose and pectin, modified starch (EUDRAGUARD^{®} Natural) and alginate and/or pectin, shellac and alginate and/or pectin, shellac and inulin, whey protein and gums (such as guar gum or tragacanth gum), zein and polyethylene glycol, sodium alginate and chitosan.

### In the following further components, which can be present in the functional or top coat are described

Unless explicitly state otherwise, the components are in general suitable to be used in both coating layers. The amount of the respective component is indicated in view of the total weight of the at least one polymer, contained in the respective coating layer, unless explicitly stated otherwise.

### Glidants

Glidants usually have lipophilic properties. They prevent agglomeration of cores during film formation of the film forming polymers.

The at least one glidant is preferably selected from silica, for example commercially available under the tradenames RXCIPIENTS^{®} GL100 or RXCIPIENTS^{®} GL200, ground silica, fumed silica, kaolin calcium silicate, magnesium silicate, colloidal silicone dioxide, talc, stearate salts like calcium stearate, magnesium stearate, zinc stearate, sodium stearyl fumarate, starch, stearic acid, preferably talc, magnesium stearate, colloidal silicon dioxide und glycerol monostearate or mixtures thereof, more preferred glycerol monostearate and talc or mixtures thereof.

Standard proportions for use of glidants in the coating layer range between 0.5 and 100 % by weight, preferably 3 to 75 % by weight, more preferably 5 to 50 % by weight, most preferably 5 to 30 % by weight, relative to the total weight of the at least one polymer.

### Emulsifiers

In general, all known emulsifiers are suitable. Preferred are non-ionic emulsifier, in particular emulsifier having an HLB > 10 or HLB > 12. The HBL Value can be determined according to Griffin, William C. (1954), "Calculation of HLB Values of Non-Ionic Surfactants" (PDF), Journal of the Society of Cosmetic Chemists, 5 (4): 249-56.

The at least one emulsifier is preferably selected from polyglycosides, alcohols, sugar and sugar derivatives, polyethers, amines, polyethylene derivatives, alkyl sulfates (e.g. sodium dodecyl sulfate), alkyl ether sulfates, dioctyl sodium sulfosuccinate, polysorbates (e.g. polyoxyethylene (20) sorbitan monooleate), nonylphenol ethoxylates (nonoxynol-9) and mixtures thereof.

The at least one emulsifier is preferably selected from alkyl polyglycosides, decyl glucoside, decyl polyglucose, lauryl glucoside, octyl glucoside, N-octyl beta-D-thioglucopyranoside, cetostearyl alcohol, cetyl alcohol, stearyl alcohol, polyoxyethylene cetostearyl alcohol, cetylstearyl alcohol, oleyl alcohol, polyglyceryl-6-dioleate, glyceryl stearate citrate, polyglyceryl-3 caprate, polyglyceryl-3 diisostearate, glyceryl isostearate, polyglyceryl-4-isostearate, glyceryl monolinoleate, dicaprylyl carbonate, alcohol polyglycol ether, polyethylenglycolether of cetearylalcohols (n=20), polyethylene glycol-6 stearate, glycol stearate, polyethylene glycol-32 stearate, polyethylene glycol-20 stearate, fatty alcohol polyglycol ether, polyethylene glycol-4 laurate, polyethylene glycol isocetyl ether (n=20), polyethyleneglycol-32 (Mw 1500 g/mol) mono- and diesters of lauric acid (C12), nonaethylene glycol, polyethylene glycol nonylphenyl ether, octaethylene glycol monododecyl ether, pentaethylene glycol monododecyl ether, polyethylene glycol macrocetyl ether, polyethylene glycol esters of palmitic (C16) or stearic (C18) or caprylic acids, polyoxyethylene fatty ether derived from stearyl alcohols like BRIJ S2, polyoxyethylene oxypropylene stearate, macrogol stearyl ether (20), diethylaminoethyl stearate, polyethylene glycol stearate, sucrose distearate, sucrose tristearate, sorbitan monostearate, sorbitan tristearate, mannide monooleate, octaglycerol monooleate, sorbitan dioleate, polyricinoleate, polysorbate like polysorbate 20 and Polyoxyethylene (20) sorbitan monooleate (polysorbate 80), sorbitan, sorbitan monolaurate, sucrose cocoate, glycereth-2 cocoate, ethylhexyl cocoate, polypropylene glycol -3 benzyl ether myristate, sodium myristate, gold sodium thiomalate, polyethylene glycol 8 laurate, polyethylene-4 dilaurate, from α-Hexadecyl-ω-hydroxypoly(oxyethylene), cocamide diethanolamine, N-(2-hydroxyethyl)dodecanamide, octylphenoxypolyethoxyethanol, maltoside, 2,3-Dihydroxypropyl dodecanoate, 3-[(3R,6R,9R,12R,15S,22S,25S,30aS)-6,9,15,22-Tetrakis(2-amino-2-oxoethyl)-3-(4-hydroxybenzyl)-12-(hydroxymethyl)-18-(11-methyltridecyl)-1,4,7,10,13,16,20,23,26-nonaoxotriacontahydropyrrolo[1,2-g][1,4,7,10,13,16,19,22,25]nonaazacyclooctacosin-25-yl]propenamide, 2-{2-[2-(2-{2-[2-(2-{2-[2-(4-nonylphenoxy)ethoxy]ethoxy}ethoxy)ethoxy]ethoxy}ethoxy)ethoxy]ethoxy}ethanol, oxypolyethoxydodecane, poloxamers like poloxamer 188 (Pluronic F-68) and poloxamer 407, propylene glycol monocaprylate, type I (Capryol PGMC), polyethoxylated tallow amine, polyglycerol, polyoxyl 40 hydrogenated castor oil, surfactin, 2-[4-(2,4,4-trimethylpentan-2-yl)phenoxy]ethanol, carbomer, sodium carbomer, carboxymethylcellulose calcium, carrageenan, cholesterol, deoxycholic acid, phospholipids like egg phospholipids, gellan gum, lanolin, capric acid, waxes like Polawax NF, Polawax A31 or Ceral PW, ester gum, dea-cetyl phosphate, soya lecithin, sphingomyelins, sodium phosphate, sodium lauroyl lactylate, lanolin, Oxirane methyl-polymer with oxirane monobutyl ether, 1,2-dierucoylphosphatidylcholine, dimethicone end-blocked with an average of 14 moles of propylene oxide, laurylmethicone copolyol, lauroglycol 90, white mineral oil like Amphocerine KS, dispersion of acrylamide/sodium acryloyldimethyl taurate copolymer in isohexadecane, and sodium polyacrylate or mixtures thereof. Preferred are macrogol stearyl ether (20) and polysorbate 80.

In one embodiment less than 3 wt.-%, preferably 1.5 wt.-% of at least one emulsifier based on the total weight of the at least one polymer is present or essentially no or no emulsifier is present.

At least one emulsifier is contained in the top coat.

### Functional or top coating layer

The functional or top coating layer may comprise 10 % or more, 20 % or more, 30 % or more, 40 % or more, 50 % or more, 60 % or more, 70 % or more, 80 % or more, 90 % or more by weight or 95 % or more by weight of the at least one polymer. The coating layer may comprise 10 - 100, 10 - 90, 12 - 80, 15 - 80, 18 - 80, 20 - 80 or 40 to 80 % by weight of the at least one polymer.

The top coating layer is located on the functional coating layer, comprising the at least one polymer as disclosed. A top coat is also preferably water-soluble or essentially water-soluble. A top coat may have the function of colouring the pharmaceutical or nutraceutical form or protecting from environmental influences for instance from moisture during storage.

### Amount and thickness of the functional and top coating layer

In order to ensure influx prevention and processability of the final capsule in an industrial filling machine, it has been found that the total coating amount is required to be 2.0 to 10 mg/cm² and the coating amount of the top coat is at most 40% of the coating amount of the functional coat.

For a hard shell capsule of size #0, the amount of the coating layer should not be too high. If the amount of coating layer applied is too high this may result in difficulties to process the polymer-coated pre-locked hard shell capsules subsequently in a capsule-filling machine. If the amount of coating layer is less than 5 mg/cm², for instance 2 to 4 mg/cm² usually no problem with standard capsule-filling machines without modification will occur. In the range from 4 and up to about 8 mg/cm² capsule-filling machines can still be used, however the forms for the bodies and the caps should be adjusted to be somewhat wider. Such an adjustment can be easily performed by a mechanical engineer. Thus capsule-filling machines can be advantageously used within a range of an amount of coating layer from about 3 to about 8 mg/cm².

For a hard shell capsule of size #1, the amount of the coating layer should not be too high. If the amount of coating layer applied is too high this may result in difficulties to process the polymer-coated pre-locked hard shell capsules subsequently in a capsule-filling machine. If the amount of coating layer is less than 4 mg/cm², for instance 2 to 3.5 mg/cm² usually no problem with standard capsule-filling machines without modification will occur. In the range from 3.5 and up to about 8 mg/cm² capsule-filling machines can still be used, however the forms for the bodies and the caps should be adjusted to be somewhat wider. Such an adjustment can be easily performed by a mechanical engineer. Thus capsule-filling machines can be advantageously used within a range of an amount of coating layer from about 3 to about 8 mg/cm².

For a hard shell capsule of size #3, the amount of the coating layer should not be too high. If the amount of coating layer applied is too high this may result in difficulties to process the polymer-coated pre-locked hard shell capsules subsequently in a capsule-filling machine. In the range from 2 and up to about 6 mg/cm² capsule-filling machines can still be used, however the forms for the bodies and the caps should be adjusted to be somewhat wider. Such an adjustment can be easily performed by a mechanical engineer. Thus capsule-filling machines can be advantageously used within a range of an amount of coating layer from about 3 to about 6 mg/cm².

If the amount of coating layer applied is too high there will be also an assembly of too much coating layer at the rim of the cap where the gap between body and cap is in the pre-locked state. This may result after drying in fissures of the coating layer when the coated pre-locked hard shell capsule is opened manually or in a machine. The fissures may result in a later leakage of the capsule. Finally, a too thick coating may result in difficulties or make it impossible to close the opened coated hard shell capsule to the final-locked state since the coating layer is thicker than the gap in the overlapping area between the body and the cap.

As a rough rule the coating layer on the hard shell capsule can be applied in an amount (= a total weight gain) of 0.7 to 20, 1.0 - 18, 2 to 10, 4 to 8, 1.0 to 8, 1.5 to 5.5, 1.5 to 4mg/cm².

As a rough rule the coating layer on the hard shell capsule may have an average thickness of about 5 to 100, 10 to 50, 15 to 75 µm.

As a rough rule the coating layer on the hard shell capsule can be applied in an amount of 5 to 50, preferably 8 - 40 % dry weight in relation to the weight of the pre-locked capsule.

With this guidance a skilled person will be able to adjust the amounts of the coating layer in a range between too low and too high.

### Biologically active ingredient

The biologically active ingredient is preferably a pharmaceutical active ingredient and/or a nutraceutical active ingredient and/or a cosmetically active ingredient. Even though it is possible that certain biologically active ingredients are contained in the respective coating layers, it is preferred that the biologically active ingredient is contained in the fill-in. In particular, if the biologically active ingredient is a liposome, lipid nanoparticle or nucleic acid, the biologically active ingredient is only contained in the fill-in.

### Pharmaceutical or nutraceutical active ingredients

The invention is preferably useful for immediate, delayed release or sustained release formulated pharmaceutical or nutraceutical dosage forms with a fill-in of pharmaceutical or nutraceutical active ingredients.

Suitable therapeutic and chemical classes of pharmaceutical active ingredients which members can be used as fill-in for the described polymer-coated hard shell capsules are for instance: analgesics, antibiotics or anti-infectives, antibodies, antiepileptics, antigens from plants, antirheumatics, benzimidazole derivatives, beta-blocker, cardiovascular drugs, chemotherapeutics, CNS drugs, digitalis glycosides, gastrointestinal drugs, e.g. proton pump inhibitors, enzymes, hormones, liquid or solid natural extracts, oligonucleotides, peptide, hormones, proteins, therapeutic bacteria, peptides, proteins (metal)salt i.e. aspartates, chlorides, urology drugs, lipid nanoparticles, liposomes, polymeric nanoparticles, vaccines. In a preferred embodiment at least one liposome or lipid nanoparticle is contained.

In a preferred embodiment the pharmaceutically active ingredient is a lipid nanoparticle, liposome or a nucleic acid, more preferably a nucleic acid agent can be DNA, RNA, or combinations thereof. In some embodiments, a nucleic acid agent can be an oligonucleotide and/or polynucleotide. In some embodiments, a nucleic acid agent may be an oligonucleotide and/or modified oligonucleotide (including, but not limited to, modifications through phosphorylation); an antisense oligonucleotide and/or modified antisense oligonucleotide (including, but not limited to, modifications through phosphorylation). In some embodiments, a nucleic acid agent can comprise cDNA and/or genomic DNA. In some embodiments, a nucleic acid agent can comprise non-human DNA and/or RNA (e.g., viral, bacterial, or fungal nucleic acid sequences). In some embodiments, a nucleic acid agent can be a plasmid, cosmid, gene fragment, artificial and/or natural chromosome (e.g., a yeast artificial chromosome), and/or a part thereof. In some embodiments, a nucleic acid agent can be a functional RNA (e.g., mRNA, a tRNA, an rRNA and/or a ribozyme). In some embodiments, a nucleic acid agent can be an RNAi-inducing agent, small interfering RNA (siRNA), short hairpin RNA (shRNA), and/or microRNA (miRNA). In some embodiments, a nucleic acid agent can be a peptide nucleic acid (PNA). In some embodiments, a nucleic acid agent can be a polynucleotide comprising synthetic analogues of nucleic acids, which may be modified or unmodified. In some embodiments, a nucleic acid agent can comprise various structural forms of DNA including single-stranded DNA, double-stranded DNA, supercoiled DNA and/or triple -helical DNA; Z-DNA; and/or combinations thereof. Further suitable nucleic acids are for example disclosed in WO 2012103035 A1.

Further examples of drugs that can be used as fill-in for the described polymer-coated hard shell capsules are for instance acamprosat, aescin, amylase, acetylsalicylic acid, adrenalin, 5-amino salicylic acid, aureomycin, bacitracin, balsalazine, beta carotene, bicalutamid, bisacodyl, bromelain, bromelain, budesonide, calcitonin, carbamacipine, carboplatin, cephalosporins, cetrorelix, clarithromycin, chloromycetin, cimetidine, cisapride, cladribine, clorazepate, cromalyn, 1-deaminocysteine-8-D-arginine-vasopressin, deramciclane, detirelix, dexlansoprazole, diclofenac, didanosine, digitoxin and other digitalis glycosides, dihydrostreptomycin, dimethicone, divalproex, drospirenone, duloxetine, enzymes, erythromycin, esomeprazole, estrogens, etoposide, famotidine, fluorides, garlic oil, glucagon, granulocyte colony stimulating factor (G-CSF), heparin, hydrocortisone, human growth hormon (hGH), ibuprofen, ilaprazole, insulin, Interferon, Interleukin, Intron A, ketoprofen, lansoprazole, leuprolidacetat lipase, lipoic acid, lithium, kinin, memantine, mesalazine, methenamine, milameline, minerals, minoprazole, naproxen, natamycin, nitrofurantion, novobiocin, olsalazine, omeprazole, orothates, pancreatin, pantoprazole, parathyroid hormone, paroxetine, penicillin, perprazol, pindolol, polymyxin, potassium, pravastatin, prednisone, preglumetacin progabide, pro-somatostatin, protease, quinapril, rabeprazole, ranitidine, ranolazine, reboxetine, rutosid, somatostatin streptomycin, subtilin, sulfasalazine, sulphanilamide, tamsulosin, tenatoprazole, thrypsine, valproic acid, vasopressin, vitamins, zinc, including their salts, derivatives, polymorphs, isomorphs, or any kinds of mixtures or combinations thereof.

It is evident to a skilled person that there is a broad overlap between the terms pharmaceutical and nutraceutical active ingredients, excipients and compositions respectively a pharmaceutical or a nutraceutical dosage form. Many substances listed as nutraceuticals may also be used as pharmaceutical active ingredients. Depending on the specific application and local authority legislation and classification, the same substance can be listed as a pharmaceutical or a nutraceutical active ingredient respectively a pharmaceutical or a nutraceutical composition or even both.

Nutraceuticals are well known to the skilled person. Nutraceuticals are often defined as extracts of foods claimed to have medical effects on human health. Thus, nutraceutical active ingredients may display pharmaceutical activities as well: Examples for nutraceutical active ingredients can be resveratrol from grape products as an antioxidant, soluble dietary fiber products, such as psyllium seed husk for reducing hypercholesterolemia, broccoli (sulphane) as a cancer preservative, and soy or clover (isoflavonoids) to improve arterial health. Thus, it is clear that many substances listed as nutraceuticals may also be used as pharmaceutical active ingredients.

Typical nutraceuticals or nutraceutical active ingredients that can be used as fill-in for the described polymer-coated hard shell capsules may also include probiotics and prebiotics. Probiotics are living microorganisms believed to support human or animal health when consumed. Prebiotics are nutraceuticals or nutraceutical active ingredients that induce or promote the growth or activity of beneficial microorganisms in the human or animal intestine.

Examples for nutraceuticals are resveratrol from grape products, omega-3-fatty acids or pro-anthocyanines from blueberries as antioxidants, soluble dietary fiber products, such as psyllium seed husk for reducing hypercholesterolemia, broccoli (sulphane) as a cancer preservative, and soy or clover (isoflavonoids) to improve arterial health. Other nutraceuticals examples are flavonoids, antioxidants, alpha-linoleic acid from flax seed, beta-carotene from marigold petals or antocyanins from berries. Sometimes the expression neutraceuticals or nutriceuticals are used as synonyms for nutraceuticals.

Preferred biologically active ingredients are metoprolol, mesalamine and omeprazole.

### Additives

Additives according to the present invention are preferably excipients, which are well known to a skilled person and often formulated along with the biologically active ingredient contained in the coated hard shell capsule and/or with a polymer coating layer of the hard shell capsule as disclosed and claimed herein. All excipients used must be toxicologically safe and be used in pharmaceuticals or nutraceuticals without risk for patients or consumers.

The dosage form may comprise excipients, preferably pharmaceutical or nutraceutical acceptable excipients, selected from the group of antioxidants, brighteners, binding agents, flavouring agents, flow aids, fragrances, penetration-promoting agents, pigments, pore-forming agents or stabilizers or combinations thereof. The pharmaceutically or nutraceutically acceptable excipients can be comprised in the core and/or in the coating layer comprising the polymer as disclosed. A pharmaceutical or nutraceutical acceptable excipient is an excipient, which is allowed to be used for the application in the pharmaceutical or nutraceutical field.

The functional or top coating layer may comprise up to 90, up to 80, up to 70, up to 50, up to 60, up to 50, up to 40, up to 30, up to 20, up to 10, up to 5 % up to 3 %, up to 1 % by weight or not any (0 %) additives at all, respectively pharmaceutically or nutraceutically acceptable excipients, based on the total weight of the at least one polymer.

### Plasticizers

The polymer coating of the hard shell capsule may comprises one or more plasticizers. Plasticizers achieve through physical interaction with a polymer a reduction in the glass transition temperature and promote film formation, depending on the added amount. Suitable substances usually have a molecular weight of between 100 and 20,000 g/mol and comprise one or more hydrophilic groups in the molecule, e.g. hydroxyl, ester or amino groups.

Examples of suitable plasticizers are alkyl citrates, alkyl phthalates, alkyl sebacates, diethyl sebacate, dibutyl sebacate, polyethylene glycols, and polypropylene glycols. Preferred plasticizers are triethyl citrate (TEC), acetyl triethyl citrate (ATEC), diethyl sebacate, dibutyl sebacate (DBS), polyethylene glycols, and polypropylene glycols or mixtures thereof.

Addition of the plasticizers to the formulation can be carried out in a known manner, directly, in aqueous solution or after thermal pre-treatment of the mixture. It is also possible to employ mixtures of plasticizers. The polymer coating of the hard shell capsule may comprise one or more plasticizers, preferably up to 60, up to 30, up to 25, up to 20, up to 15, up to 10, up to 5, less than 5% by weight, calculated on the at least one polymer, of a plasticizer or any (0 %) plasticizer at all can be comprised.

The top coat comprises at least one plasticizer.

### Fillers

Standard fillers are usually added to the inventive formulation during processing to coating and binding agents. The quantities introduced and the use of standard fillers in pharmaceutical coatings or over layers is familiar to those skilled in the art. Examples of standard fillers are release agents, pigments, stabilizers, antioxidants, pore-forming agents, penetration-promoting agents, brighteners, fragrances or flavoring agents. They are used as processing adjuvants and are intended to ensure a reliable and reproducible preparation process as well as good long-term storage stability, or they achieve additional advantageous properties in the pharmaceutical form. They are added to the polymer formulations before processing and can influence the permeability of the coatings. This property can be used if necessary, as an additional control parameter.

### Pigments

Only rarely a pigment is added in soluble form. As a rule, pigments, such as aluminum oxide or iron oxide pigments are used in dispersed form. Titanium dioxide is used as a whitening pigment. Standard proportions for use of pigments are between 10 - 200, 20 - 200 % by weight relative to the total weight of the at least one polymer in the coating layer. Proportions up to 200 % by weight based on the total weight of the at least one polymer can be easily processed.

In a particularly advantageous embodiment, the pigment is used in the top coat. Application takes place in the form of powder or by spraying from aqueous suspension with 5 to 35% (w/w) solid content. The necessary concentration is lower than for incorporation into the polymer layer and amounts to 0.1 to 2% by weight relative to the weight of the pharmaceutical form.

### Optional sub coats

Optionally the hard shell capsule can be additionally coated with a sub coat.

A sub coat can be located between capsule and the functional coating layer, comprising at least one polymer as disclosed above. A sub coat has essentially no influence on the active ingredient release characteristics but may for instance improve the adhesion of the polymer coating layer. A sub coat is preferably essentially water-soluble, for instance it may consist of substances like HPMC as a film former. The average thickness of a sub coat layer is usually very thin, for example not more than 15 µm, preferably not more than 10 µm (0.1 - 1.0 mg/cm²). A sub coat has not necessarily to be applied on the hard shell capsule in the pre-locked state.

### Process for preparing a coated hard shell capsule

Described is a process for preparing a polymer-coated hard shell capsule, suitable as container for pharmaceutical or nutraceutical biologically active ingredients, wherein the hard shell capsule comprises a body and a cap, wherein in the closed state the cap overlaps the body either in a pre-locked state or in a final-locked state, wherein the hard shell capsule is provided in the pre-locked state and coated, preferably spray-coated, with a coating solution, suspension or dispersion according to the present invention to create a functional coating layer, then optionally dried and coated, preferably spray-coated, with a coating solution, suspension or dispersion according to the present invention to create a top coating layer, which covers the outer surface of the hard shell capsule in the pre-locked state.

In a further process step the pre-locked hard shell capsule can be provided with a fill comprising a pharmaceutical or a nutraceutical biologically active ingredient and is closed to the final-locked state.

In such a further process step the polymer-coated hard shell capsule in the pre-locked state can be opened, filled with a fill comprising at least one biologically active ingredient, and is closed in the final-locked state. This further process step is preferably performed in that the coated hard shell capsule in the pre-locked state is provided to a capsule-filling machine, which performs the opening, filling with a fill comprising at least one biologically active ingredient and closing of the polymer-coated hard shell capsule to the final-locked state.

This further process step results in a final-locked polymer-coated hard shell capsule, which is a container for at least one biologically active ingredient. The final-locked polymer-coated hard shell capsule, which as a container for at least one biologically active ingredient is preferably a pharmaceutical or nutraceutical dosage form.

The pharmaceutical or nutraceutical dosage form preferably comprises a polymer-coated hard shell capsule in the final-locked state containing a fill comprising at least one biologically active ingredient, wherein the polymer-coated hard shell capsule comprises a coating layer according to the invention, where the coating layer covers the outer surface area of the capsule in the pre-locked state but not the overlapping area where the cap covers the body in the pre-locked state.

A coating suspension comprising the at least one polymer can contain an organic solvent, for instance acetone, iso-propanol or ethanol. The concentration of dry weight material in the organic solvent can be about from 5 to 50 % by weight of polymer. A suitable spraying concentration can be about 5 to 25 % by dry weight.

A coating suspension can be the dispersion of the at least one polymer in an aqueous medium, for instance water or a mixture of 80 % by weight or more of water and 20 % or less by weight of water-soluble solvents, such as acetone or isopropanol. A suitable concentration of dry weight material in the aqueous medium can be from about 5 to 50 % by weigh. A suitable spraying concentration can be about 5 to 25 % by dry weight.

The spray coating is preferably performed by spraying the coating solution or dispersion onto the pre-locked capsules in a drum coater or in a fluidized bed coating equipment.

### Process for preparing a fill for the dosage form

Suitable processes for preparing the fill for the pharmaceutical or nutraceutical dosage form are well known to a skilled person. A suitable process for preparing the fill for the pharmaceutical or nutraceutical dosage form as disclosed herein can be by forming a core comprising the biologically active ingredient in the form of pellets by direct compression, compression of dry, wet or sintered granules, by extrusion and subsequent rounding off, by wet or dry granulation, by direct pelleting or by binding powders onto active ingredient-free beads or neutral cores or active ingredient-containing particles or pellets and optionally by applying coating layers in the form of aqueous dispersions or organic solutions in spray processes or by fluidized bed spray granulation.

### Capsule filling machine

The polymer-coated hard-shell capsule is provided in the pre-locked state to a capsule-filling machine, which performs the steps of separating the body and the cap, filling the body with the fill and rejoining the body and the cap in the final-locked state.

The capsule filling machine used can be a capsule filling machine, preferably a fully automated capsule filling machine, that is capable to produce filled and closed capsules at a speed with an output of 1,000 or more filled and finally closed capsules per hour. Capsule filling machines, preferably fully automated capsule filling machines, are well known in the art and commercially available from several companies. A suitable capsule filling machine as used in the examples can be for instance ACG, model AFT Lab.

The capsule filling machine used can be preferably operated at a speed with an output of 1,000 or more, preferably 10,000 or more, 30,000 or more, 100,000 or more, 10,000 up to 500,000, filled and finally closed capsules per hour. In general, an output of less than 10,000 capsules per hour is considered to be lab scale, an output of less than 30,000 is considered to be pilot scale.

### Capsule filling machine general operations

Before the capsule filling process, the capsule filling machine is provided with a sufficient number or amount of pre-coated hard-shell capsules in the pre-locked state. The capsule filling machine is also provided with sufficient amounts of fill to be filled in during operation.

The hard-shell capsules in the pre-locked state may fall by gravity into feeding tubes or chutes. The capsules can be uniformly aligned by mechanically gauging the diameter differences between the cap and the body. The hard-shell capsules are then usually fed, in proper orientation, into a two-section housing or brushing.

The diameter of the upper bushing or housing is usually larger than the diameter of the capsule body bushing; thus, the capsule cap can be retained within an upper bushing while the body is pulled into a lower bushing by vacuum. Once the capsule is opened/ the body and the cap are separated, the upper and lower housing or bushing are separated to position the capsule body for filling.

The open capsule body is then filled with the fill. Various types of filling mechanisms can be applied, with respect to the different fillings such as granules, powders, pellets or mini-tablets. Capsule filling machines in general employ a variety of mechanisms to handle the various dosage ingredients as well as various numbers of filling stations. The dosing systems are usually based on volumetric or amounts of fills governed by the capsule size and capacity of the capsule body. The empty capsule manufacturers usually provide reference tables that indicate the volume capacity of their capsule body and the maximum fill weight for different capsule sizes based on the density of the fill material. After the filling, the body and the cap are rejoined by the machine in the final-locked state or position.

### Use / method of use / method steps

The process for preparing a polymer-coated hard shell capsule suitable as described herein can be understood as a method of use of a hard shell capsule comprising a body and a cap, wherein in the closed state the cap overlaps the body either in a pre-locked state or in a final-locked state, for preparing a polymer-coated hard shell capsule, suitable as container for pharmaceutical or nutraceutical biologically active ingredients, comprising the steps of
a) providing the hard shell capsule is provided in the pre-locked state and
b) spray-coating with a first and second coating solution, suspension or dispersion comprising a polymer or a mixture of polymers to create a functional and a top coating layer which covers the outer surface of the hard shell capsule in the pre-locked state.

The spray-coating can be preferably applied by using a drum coater equipment or a fluidized bed coating equipment. A suitable product temperature during the spray-coating process can be in the range from about 15 to 40, preferably from about 20 to 35 °C. A suitable spray rate can be in the range from about 0.3 to 17.0, preferably 0.5 to 14 [g/min/kg]. After spray-coating a drying step is included.

The polymer-coated hard shell capsule in the pre-locked state can be opened in a step c), filled with a fill comprising a pharmaceutical or a nutraceutical biologically active ingredient in a step d), and is then closed in a step e) to the final-locked state.

Steps c) to e) can be performed manually or preferably supported by a suitable equipment, for instance a capsule-filling machine. Preferably, the coated hard shell capsule in the pre-locked state is provided to a capsule-filling machine, which performs the opening step c), the filling with a fill comprising a pharmaceutical or a nutraceutical biologically active ingredient in step d) and the closing of the capsule to the final-locked state in step e).

The selection of the processes in all their generic or specific features and embodiments as disclosed herein can be combined without restriction with any other generic or specific selections of materials or numerical features and embodiments as disclosed herein, such as polymers, capsule materials, capsule sizes, coating thicknesses, biologically active ingredients and any other embodiments as disclosed.

### Pharmaceutical or nutraceutical dosage form

Disclosed is a pharmaceutical or nutraceutical dosage form comprising a polymer-coated hard shell capsule in the final-locked state containing a fill comprising a pharmaceutical or nutraceutical biologically active ingredient, wherein the polymer-coated hard shell capsule comprises a coating layer comprising a polymer or a mixture of polymers, where the coating layer covers the outer surface area of the capsule in the pre-locked state. Since the outer surface area of the capsule in the pre-locked state is larger than outer surface area of the capsule in the final-locked state a part of the polymer coating layer is hidden or enclosed between the body and the cap of the hard shell capsule, which provides an efficient sealing.

### Items

In particular, the present invention refers to:
1. Process for preparing a polymer-coated hard shell capsule comprising at least a functional coat and a top coat, suitable as container for pharmaceutical or nutraceutical biologically active ingredients, wherein the hard shell capsule comprises a body and a cap, wherein in the closed state the cap overlaps the body either in a pre-locked state or in a final-locked state, wherein the hard shell capsule is provided in the pre-locked state and
   is coated, preferably spray-coated,
   with a first coating solution, suspension or dispersion comprising or consisting of
      a1) at least one polymer;
      b1) optionally at least one glidant;
      c1) optionally at least one emulsifier;
      d1) optionally at least one plasticizer;
      e1) optionally at least one biologically active ingredient; and
      f1) optionally at least one additive, different from a1) to e1);
   to obtain the functional coat of the hard shell capsule in the pre-locked state; and thereafter is coated with a second coating solution, suspension or dispersion, which is different from the first coating solution, suspension or dispersion, comprising or consisting of
      a2) at least one polymer;
      b2) optionally at least one glidant;
      c2) at least one emulsifier;
      d2) at least one plasticizer;
      e2) optionally at least one biologically active ingredient; and
      f2) optionally at least one additive, different from a2) to e2);
   to obtain the top coat of the hard shell capsule in the pre-locked state, wherein
   the total coating amount is 2 to 10 mg/cm², preferably 2.2 to 9 mg/cm²; more preferably 2.5 to 8 mg/cm²; and
   the coating amount of the top coat is at most 40%, at most 30 %, preferably at most 28% of the coating amount of the functional coat.
2. Process according to item 1, wherein the base material of the body and the cap is selected from hydroxypropyl methyl cellulose, starch, gelatin, pullulan and a copolymer of a C1- to C4-alkylester of (meth)acrylic acid and (meth)acrylic acid, preferably is hydroxypropyl methyl cellulose.
3. Process according to item 1 or 2, wherein the at least one polymer a1) and/or a2), preferably a1), is selected from at least one anionic polymer or at least one (meth)acrylate copolymer, preferably at least one anionic (meth)acrylate copolymer; more preferably having a glass transition temperatures *T_{gm}* of 125 °C or less.
4. Process according to item 1 or 2, wherein the at least one polymer a1) and/or a2), preferably a1), is
   i) a Core-Shell polymer, which is a copolymer obtained by a two stage emulsion polymerization process with a core with 70 to 80 % by weight, comprising polymerized units of 65 to 75 % by weight of ethyl acrylate and 25 to 35 % by weight of methyl methacrylate, and a shell with 20 to 30 % by weight, comprising polymerized units of 45 to 55 % by weight ethyl acrylate and 45 to 55 % by weight methacrylic acid; or
   ii) an anionic polymer obtained by polymerizing 25 to 95 % by weight C1- to C12-alkyl esters of acrylic acid or of methacrylic acid and 75 to 5% by weight (meth)acrylate monomers with an anionic group; or
   iii) a cationic (meth)acrylate copolymer obtained by polymerizing C1- to C4-alkyl esters of acrylic or of methacrylic acid and an alkyl ester of acrylic or of methacrylic acid with a tertiary or a quaternary ammonium group in the alkyl group; or
   iv) a (meth)acrylate copolymer obtained by polymerizing methacrylic acid and ethyl acrylate, methacrylic acid and methyl methacrylate, ethyl acrylate and methyl methacrylate or methacrylic acid, methyl acrylate and methyl methacrylate; or
   v) a (meth)acrylate copolymer obtained by polymerizing 40 to 60 % by weight of methacrylic acid and 60 to 40 % by weight of ethyl acrylate; or
   vi) a (meth)acrylate copolymer obtained by polymerizing 60 to 80 % of ethyl acrylate and 40 to 20 % by weight of methyl methacrylate; or
   vii) a (meth)acrylate copolymer obtained by polymerizing 5 to 15 % by weight methacrylic acid, 60 to 70 % by weight of methyl acrylate and 20 to 30 % by weight methyl methacrylate; or mixtures thereof.
5. Process according to item 1 or 2, wherein the at least one polymer a1) and/or a2), preferably a1), is a mixture of
   i) a (meth)acrylate copolymer obtained by copolymerizing 40 to 60 % by weight of methacrylic acid and 60 to 40 % by weight of ethyl acrylate and a (meth)acrylate copolymer obtained by polymerizing 60 to 80, preferably 60 to 78,% by weight of ethyl acrylate and 40 to 20, preferably 20 to 38, % by weight of methyl methacrylate preferably at a ratio from 10:1 to 1:10 by weight and optionally up to 2 % by weight of (meth) acrylic acid; or
   ii) a (meth)acrylate copolymer obtained by copolymerizing 5 to 15 % by weight methacrylic acid, 60 to 70 % by weight of methyl acrylate and 20 to 30 % by weight methyl methacrylate and a (meth)acrylate copolymer obtained by copolymerizing 40 to 60 % by weight of methacrylic acid and 60 to 40 % by weight of ethyl acrylate preferably at a ratio from 1:1 to 5:1 by weight.
6. Process according to item 1 or 2, wherein the at least one polymer a1) and/or a2), preferably a2) is selected from at least one anionic cellulose, ethyl cellulose or starch comprising at least 35 % by weight amylose; more preferably is a methylcellulose and/or a hydroxypropyl methylcellulose.
7. Process according to item 1 or 2, wherein the at least one polymer a1) and/or a2), preferably a2) is selected from celluloses, like hydroxyethyl cellulose (HEC), hydroxypropyl cellulose (HPC), hydroxypropyl methyl cellulose (HPMC), hydroxyethyl methyl cellulose (HEMC), ethyl cellulose (EC), methyl cellulose (MC), cellulose esters, cellulose glycolates, polyethylene glycols, polyethylene oxides, polyvinyl pyrrolidone, polyvinyl acetate, polyvinyl alcohol, or a mixture thereof, more preferably hydroxypropyl cellulose (HPC),hydroxypropyl methyl cellulose, polyvinyl alcohol or a mixture thereof.
8. Process according to any of the preceding items, wherein at least one glidant is present in the first and/or second coating solution, suspension or dispersion,
   preferably the at least one glidant
   i) is present in an amount of 3 to 75 % by weight, based on the total weight of the at least one polymer and/or
   ii) is selected from silica, ground silica, fumed silica, kaolin calcium silicate, magnesium silicate, colloidal silicone dioxide, talc, stearate salts, sodium stearyl fumarate, starch, stearic acid or mixtures thereof, preferably talc, magnesium stearate, colloidal silicon dioxide and glycerol monostearate or mixtures thereof, more preferred glycerol monostearate, talc and mixtures thereof.
9. Process according to any of the preceding items, wherein at least one emulsifier is present in the first coating solution, suspension or dispersion, wherein the at least one emulsifier preferably
   i) is present in an amount of less than 3 % by weight, preferably less than 1.5 % by weight, based on the total weight of the at least one polymer; or
   ii) is present in an amount of 1.5 to 40 % by weight, based on the total weight of the at least one polymer; and/or
   iii) is a non-ionic emulsifier, preferably a non-ionic emulsifier having an HLB > 10, preferably > 12.
10. Process according to any of the preceding items, wherein the at least one emulsifier present in the second coating solution, suspension or dispersion
   i) is present in an amount of less than 3 % by weight, preferably less than 1.5 % by weight, based on the total weight of the at least one polymer; or
   ii) is present in an amount of 1.5 to 40 % by weight, based on the total weight of the at least one polymer; and/or
   iii) is a non-ionic emulsifier, preferably a non-ionic emulsifier having an HLB > 10, preferably > 12.
11. Process according to any of the preceding items, wherein at least one plasticizer is present in the first coating solution, suspension or dispersion, wherein the at least one plasticizer preferably
   i) is present in an amount of 2 to 40 % by weight, based on the total weight of the at least one polymer and/or
   ii) is selected from alkyl citrates, alkyl phthalates, and alkyl sebacates or mixtures thereof, preferably diethyl sebacate, triethyl citrate (TEC), acetyl triethyl citrate (ATEC), diethyl sebacate and dibutyl sebacate (DBS) or mixtures thereof.
12. Process according to any of the preceding items, wherein the at least one plasticizer present in the second coating solution, suspension or dispersion,
   i) is present in an amount of 2 to 40 % by weight, based on the total weight of the at least one polymer and/or
   ii) is selected from alkyl citrates, alkyl phthalates, and alkyl sebacates or mixtures thereof, preferably diethyl sebacate, triethyl citrate (TEC), acetyl triethyl citrate (ATEC), diethyl sebacate and dibutyl sebacate (DBS) or mixtures thereof.
13. Process according to any of the preceding items, wherein up to 400 % by weight, preferably up to 200 % by weight, more preferably up to 100 % by weight, on the total weight of the at least one polymer, of at least one additive are comprised in the first and/or second coating solution, suspension or dispersion; preferably selected from antioxidants, brighteners, flavouring agents, flow aids, fragrances, penetration-promoting agents, pigments, polymers different from a), pore-forming agents or stabilizers, or combinations thereof.
14. Process according to any of the preceding items, wherein the body and the cap are comprising encircling notches or dimples in the area where the cap overlaps the body, that allow the capsule to be closed by a snap-into-place mechanism either in the pre-locked state or in the final-locked state.
15. Process according to any of the preceding items, wherein the body comprises a tapered rim.
16. Process according to any of the preceding items, wherein the coating layer is applied in an amount of about 0.7 to 20 mg/cm², preferably 2 to 10, 4 to 8, 1.0 to 8, 1.5 to 5.5, or 1.5 to 4 mg/cm².
17. Process according to any of the preceding items, wherein the polymer-coated hard shell capsule in the pre-locked state is opened, filled with a fill comprising a pharmaceutical or a nutraceutical biologically active ingredient, and is closed to the final-locked state.
18. Process according to any of the preceding items, wherein the polymer-coated hard shell capsule in the pre-locked state is provided to a capsule-filling machine, which performs opening, filling with a fill comprising a pharmaceutical or a nutraceutical biologically active ingredient and closing to the final-locked state.
19. Polymer-coated hard shell capsule, obtained from a process according to any of items 1 to 18.
20. Use of the polymer-coated hard shell capsule according to item 19 for immediate, delayed or sustained release, preferably delayed release, more preferably for immediate, delayed or sustained release for intestine delivery.

### Examples

### Example 1: Moisture uptake during disintegration testing

An important criterion for the formulation of moisture or acid sensitive API's is the prevention or limitation of gastric media influx during *in-vitro* or *in-vivo* testing of delayed-release capsule formulation. Especially, for mRNA lipid nanoparticle formulations the uptake of digestive enzymes during the gastric passage is critical. For example, pepsin is found in the stomach and degrades proteins (Ball et al., Oral delivery of siRNA lipid nanoparticles: Fate in the GI tract, Scientific Reports,

(2018) 8:2178). As a key functionality of a delayed release formulation is the protection of the included active pharmaceutical excipient during gastric passage, it is important to limit the influx of gastric fluids and simulated gastric fluid during *in-vitro* or *in-vivo* testing, respectively. Therefore, the influx properties of delayed release pre-coated empty capsules have been investigated.

### Test Method:

Disintegration Test according to United State Pharmacopeia (US) 43 NF38 General Chapter <701>. The disintegration test was performed utilizing three media compositions simulating the gastric passage until release of the capsules. Therefore, three n = 3 investigations per formulations have been performed and stopped at the different test media stages to perform loss on drying testing (LOD) of the capsule content. Capsules have been filled with a powder test composition of total weight of 500 mg lactose. Capsules were gently dried with a wipe and carefully opened at the end of the disintegration test. The powder blend inside the capsules was tested for LOD.

### Tests Per Formulation:

1. 2 hours 0.1 N Hydrochloric acid followed by LOD testing (Disintegration testing without disk).
2. 2 hours 0.1 N Hydrochloric acid followed by a full change to 1 hour Phosphate Buffer pH 5.5 followed by LOD testing (Disintegration testing without disk).
3. 2 hours 0.1 N Hydrochloric acid followed by a full change to 1 hour Phosphate Buffer pH 5.5, full change to 1 hour Phosphate Buffer pH 6.8followed by detection of the disintegration time (disk only in the final stage to detect the disintegration time)

### LOD Testing

according to United State Pharmacopeia (US) 43 NF38 General Chapter <731>
Equipment: Moisture Analyzer HC 103 (Mettler -Toledo GmbH)
Sample Net Weight: 1 - 1.5 g
Stop Criterion 3: 1g/50g
Temperature: 105 °C

### Buffer Media:

Phosphate Buffer pH 5.5
65.5g potassium dihydrogen phosphate
6.5g disodium hydrogen phosphate
5I Water

### Phosphate Buffer pH 6.8

10 g potassium dihydrogen phosphate
20 g dipotassium hydrogen phosphate
85 g Sodium chloride auf
10 I Water

### Results:

**Table 1: Moisture uptake during disintegration testing**

| Formulations | Example 2 | Example 3 | Example 4 | Example 5 | Example 6 | Example 7 | Example 8 |
|---|---|---|---|---|---|---|---|
| Functional Polymers | EUDRAGIT L 30 D-55/ EUDRAGIT NM 30 D (9:1) | | | | EUDRAGIT L 30 D-55/ EUDRAGIT FS 30 D (1:1) | | EUDRAGIT L 30 D-55/ EUDRAGIT FS 30 D (7:3) |
| Ratio 1^{st} / 2^{nd} layer | 78/22 | 84/16 | 89/11 | 82/18 | 89/11 | 95/5 | 95/5 |
| Total solid weight gain (total coating amount) | 5.1 mg/cm² | 5.19 mg/cm² | 5.3 mg/cm² | 3.3 mg/cm² | 5.5 mg/cm² | 5.5mg/cm² | 5.5mg/cm² |
| LOD untreated | 0.65 % | 0.65 % | 0.30 % | 0.65 % | 0.30 % | 0.50 % | 0.34 % |
| LOD after Test 1 | 1.04 % | 2.96 % | 2.41 % | 0.70 % | 0.33% | 0.76 % | 0.90 % |
| Average Media Uptake | 0.39 % | 2.31 % | 2.11 % | 0.05% | 0.03 % | 0.26 % | 0.56 % |
| LOD after Test 2 | 3.92 % | > 10 % | 14.20 % | > 10 % | 2.67 % | 0.78 % | 2.37 % |
| Mean disintegration time Test 3 | 4min 27sec | 5min 32sec | 5min 41sec | 1min 31sec | 8min 55 sec | 54min 44sec | 22min 23sec |

### Example 2: (Inventive) Enteric coating of pre-locked capsules in drum coater

The functional and top coat formulations are calculated considering a surface area in pre-locked state of 545.82 mm² and a batch size of 9,000 capsules (Capsugel V-Caps Size 0).

### Functional Coating

Preparations of GMS emulsion, 40% of the water was heated up to 70-80°C. The Polysorbate 80 solution, triethyl citrate and GMS were homogenized in the heated water using a homogenizer (e. g. Ultra Turrax) for 10 minutes. The solids content was approximately 15%. The remaining 60% of water was stirred into the hot GMS emulsion by using a conventional stirrer and cooled down to room temperature while continuous stirring. Then the excipient suspension was poured slowly into the EUDRAGIT^{®} L 30 D-55 dispersion while stirring gently with a conventional stirrer. After 10 minutes gentle stirring the EUDRAGIT^{®} NM 30 D was added slowly under continuous stirring and stirred for further 15 minutes. The final coating suspension was sieved throughout a 400µm sieve and stirred during the coating process. The capsules were coated in the pre-locked state utilizing a drum coater.

**Table 2:Functional Coating**

| **Material** | **Composition** | **Solid Composition Percentage** |
|---|---|---|
| EUDRAGIT^{®} L 30 D-55 | 2.7 mg/cm² | 67.2 % |
| EUDRAGIT^{®} NM 30D | 0.3 mg/cm² | 7.5% |
| Triethyl citrate | 20.0 % on ds* | 14.9 % |
| Glycerol monostearate | 10.0 % on ds* | 7.5 % |
| Polysorbate 80 | 4.0 % on ds* | 3.0 % |
| Demineralized Water | On demand | n/a |
| | | |
| Solid content | 10.0% w/w | |
| Total solid weight gain | 4.0 mg/cm² | |
| Total applied polymer | 3.0 mg/cm² | |

| | | |
|---|---|---|
| *Quantity based on dry polymer substance [%] | | |

### Top Coating

METHOCEL^{™} VLV was thoroughly dispersed in the water while gently stirring to prevent lumping. 40% of the water was heated up to 70-80°C. The Polysorbate 80 solution, triethyl citrate and GMS were homogenized in the heated water using a homogenizer (e. g. Ultra Turrax) for 10 minutes. The solids content should be approximately 15%. The remaining 60% of water was stirred into the hot GMS emulsion by using a conventional stirrer and cooled down to room temperature while continuous stirring. Pour the suspension slowly into the METHOCEL^{™} VLV solution while stirring gently with a conventional stirrer. Pass the spray suspension through a 0.3 mm sieve. The excipient suspension was added to the polymer dispersion. The spraying suspension was gently stirred during the coating process.

**Table 3: Top Coating**

| **Material** | **Composition** | **Solid Composition Percentage** |
|---|---|---|
| METHOCEL^{™} VLV | 1 mg/cm² | 90.9 % |
| Glycerol monostearate | 5.9 % on ds* | 5.4 % |
| Triethyl citrate | 2.9 % on ds* | 2.6 % |
| Polysorbate 80 | 1.2 % on ds* | 1.1 % |
| Demineralized Water | On demand | n/a |
| | | |
| Solid content | 5% w/w | |
| Total solid weight gain | 1.1 mg/cm² | |
| Total applied polymer | 1 mg/cm² | |

| | | |
|---|---|---|
| *Quantity based on dry polymer substance [%] | | |

### Capsule Coating Process

The capsules are coated in a fully perforated side-vended pan coating system O'Hara M10. The relevant process parameters are listed in Table 4.

**Table 4: Process Parameter**

| **Parameter** | **Value** |
|---|---|
| Machine | O'Hara M10 |
| Batch size [g] | 864 |
| Nozzle bore [mm] | 1.2 |
| Internal tube diameter [mm] | 2.0 |
| Peristaltic pump | Watson Marlow |
| Atomizing pressure [bar] | 0.8 |
| Flat pattern pressure [bar] | 0.8 |
| Pan speed [rpm] | 10 |
| Inlet air volume [m³/h] | 105 - 116 |
| Inlet air temperature [°C] | 36.0-37.1 |
| Inlet air humidity [g/m³] | 4.3 - 6.9 |
| Exhaust air temperature [°C] | 25.1 - 27.8 |
| Exhaust air humidity [g/m³] | 6.4 - 9.2 |
| Product temperature [°C] | 27.4 - 29.7 |
| Spray rate [g/min/kg] | 5.2 - 8.7 |
| Process time [min] | 300 |

### Bridging Test:

The capsules were tested for bridging between body and cap. The test was performed by holding the body and gently twisting the cap of the capsule. If the cap could not be twisted without damaging the capsule, hearing or feeling a cracking and if the cap could not be twisted at all, the capsule failed, and bridging was determined. 100 capsules were tested.

### Results:

Result of this example 0/100 need high forces to sperate capsule cap and body.

### Example 3: (Inventive) Enteric coating of pre-locked capsules in drum coater

The functional and top coat formulations are calculated considering a surface area in pre-locked state of 545.82 mm² and a batch size of 9,000 capsules (Capsugel V-Caps Size 0).

### Functional Coating

Preparations of GMS emulsion, 40% of the water was heated up to 70-80°C. The Polysorbate 80 solution, triethyl citrate and GMS were homogenized in the heated water using a homogenizer (e. g. Ultra Turrax) for 10 minutes. The solids content was approximately 15%. The remaining 60% of water was stirred into the hot GMS emulsion by using a conventional stirrer and cooled down to room temperature while continuous stirring. Then the excipient suspension was poured slowly into the EUDRAGIT^{®} L 30 D-55 dispersion while stirring gently with a conventional stirrer. After 10 minutes gentle stirring the EUDRAGIT^{®} NM 30 D was added slowly under continuous stirring and stirred for further 15 minutes. The final coating suspension was sieved throughout a 400µm sieve and stirred during the coating process. The capsules were coated in the pre-locked state utilizing a drum coater.

**Table 5:Functional Coating**

| **Material** | **Composition** | **Solid Composition Percentage** |
|---|---|---|
| EUDRAGIT^{®} L 30 D-55 | 2.925 mg/cm² | 67.2 % |
| EUDRAGIT^{®} NM 30D | 0.325 mg/cm² | 7.5% |
| Triethyl citrate | 20.0 % on ds* | 14.9 % |
| Glycerol monostearate | 10.0 % on ds* | 7.5 % |
| Polysorbate 80 | 4.0 % on ds* | 3.0 % |
| Demineralized Water | On demand | n/a |
| | | |
| Solid content | 10.0% w/w | |
| Total solid weight gain | 4.36 mg/cm² | |
| Total applied polymer | 3.25 mg/cm² | |

| | | |
|---|---|---|
| *Quantity based on dry polymer substance [%] | | |

### Top Coating

METHOCEL^{™} VLV was thoroughly dispersed in the water while gently stirring to prevent lumping. 40% of the water was heated up to 70-80°C. The Polysorbate 80 solution, triethyl citrate and GMS were homogenized in the heated water using a homogenizer (e. g. Ultra Turrax) for 10 minutes. The solids content should be approximately 15%. The remaining 60% of water was stirred into the hot GMS emulsion by using a conventional stirrer and cooled down to room temperature while continuous stirring. Pour the suspension slowly into the METHOCEL^{™} VLV solution while stirring gently with a conventional stirrer. Pass the spray suspension through a 0.3 mm sieve. The excipient suspension was added to the polymer dispersion. The spraying suspension was gently stirred during the coating process.

**Table 6: Top Coating**

| **Material** | **Composition** | **Solid Composition Percentage** |
|---|---|---|
| METHOCEL^{™} VLV | 0.75 mg/cm² | 90.9 % |
| Glycerol monostearate | 5.9 % on ds* | 5.4 % |
| Triethyl citrate | 2.9 % on ds* | 2.6 % |
| Polysorbate 80 | 1.2 % on ds* | 1.1 % |
| Demineralized Water | On demand | n/a |
| | | |
| Solid content | 5% w/w | |
| Total solid weight gain | 0.83 mg/cm² | |
| Total applied polymer | 0.75 mg/cm² | |

| | | |
|---|---|---|
| *Quantity based on dry polymer substance [%] | | |

### Capsule Coating Process

The capsules are coated in a fully perforated side-vended pan coating system O'Hara M10. The relevant process parameters are listed in Table 7.

**Table 7: Process Parameter**

| **Parameter** | **Value** |
|---|---|
| Machine | O'Hara M10 |
| Batch size [g] | 864 |
| Nozzle bore [mm] | 1.2 |
| Internal tube diameter [mm] | 2.0 |
| Peristaltic pump | Watson Marlow |
| Atomizing pressure [bar] | 0.8 |
| Flat pattern pressure [bar] | 0.8 |
| Pan speed [rpm] | 10 |
| Inlet air volume [m³/h] | 103 - 117 |
| Inlet air temperature [°C] | 26.3 - 37.1 |
| Inlet air humidity [g/m³] | 4.5 - 5.5 |
| Exhaust air temperature [°C] | 24.2 - 27.7 |
| Exhaust air humidity [g/m³] | 5.9 - 8.2 |
| Product temperature [°C] | 27.2 - 30.3 |
| Spray rate [g/min/kg] | 5.0 - 8.8 |
| Process time [min] | 302 |

### Bridging Test:

The capsules were tested for bridging between body and cap. The test was performed by holding the body and gently twisting the cap of the capsule. If the cap could not be twisted without damaging the capsule, hearing or feeling a cracking and if the cap could not be twisted at all, the capsule failed, and bridging was determined. 100 capsules were tested.

### Results:

Result of this example 0/100 need high forces to sperate capsule cap and body.

### Example 4: (Inventive) Enteric coating of pre-locked capsules in drum coater

The functional and top coat formulations are calculated considering a surface area in pre-locked state of 545.82 mm² and a batch size of 9,000 capsules (Capsugel V-Caps Size 0).

### Functional Coating

Preparations of GMS emulsion, 40% of the water was heated up to 70-80°C. The Polysorbate 80 solution, triethyl citrate and GMS were homogenized in the heated water using a homogenizer (e. g. Ultra Turrax) for 10 minutes. The solids content was approximately 15%. The remaining 60% of water was stirred into the hot GMS emulsion by using a conventional stirrer and cooled down to room temperature while continuous stirring. Then the excipient suspension was poured slowly into the EUDRAGIT^{®} L 30 D-55 dispersion while stirring gently with a conventional stirrer. After 10 minutes gentle stirring the EUDRAGIT^{®} NM 30 D was added slowly under continuous stirring and stirred for further 15 minutes. The final coating suspension was sieved throughout a 400µm sieve and stirred during the coating process. The capsules were coated in the pre-locked state utilizing a drum coater.

**Table 8:Functional Coating**

| **Material** | **Composition** | **Solid Composition Percentage** |
|---|---|---|
| EUDRAGIT^{®} L 30 D-55 | 3.15 mg/cm² | 67.2 % |
| EUDRAGIT^{®} NM 30D | 0.35 mg/cm² | 7.5% |
| Triethyl citrate | 20.0 % on ds* | 14.9 % |
| Glycerol monostearate | 10.0 % on ds* | 7.5 % |
| Polysorbate 80 | 4.0 % on ds* | 3.0 % |
| Demineralized Water | On demand | n/a |
| | | |
| Solid content | 10.0% w/w | |
| Total solid weight gain | 4.7 mg/cm² | |
| Total applied polymer | 3.5 mg/cm² | |

| | | |
|---|---|---|
| *Quantity based on dry polymer substance [%] | | |

### Top Coating

METHOCEL^{™} VLV was thoroughly dispersed in the water while gently stirring to prevent lumping. 40% of the water was heated up to 70-80°C. The Polysorbate 80 solution, triethyl citrate and GMS were homogenized in the heated water using a homogenizer (e. g. Ultra Turrax) for 10 minutes. The solids content should be approximately 15%. The remaining 60% of water was stirred into the hot GMS emulsion by using a conventional stirrer and cooled down to room temperature while continuous stirring. Pour the suspension slowly into the METHOCEL^{™} VLV solution while stirring gently with a conventional stirrer. Pass the spray suspension through a 0.3 mm sieve. The excipient suspension was added to the polymer dispersion. The spraying suspension was gently stirred during the coating process.

**Table 9: Top Coating**

| **Material** | **Composition** | **Solid Composition Percentage** |
|---|---|---|
| METHOCEL^{™} VLV | 0.5 mg/cm² | 90.9 % |
| Glycerol monostearate | 5.9 % on ds* | 5.4 % |
| Triethyl citrate | 2.9 % on ds* | 2.6 % |
| Polysorbate 80 | 1.2 % on ds* | 1.1 % |
| Demineralized Water | On demand | n/a |
| | | |
| Solid content | 5% w/w | |
| Total solid weight gain | 0.6 mg/cm² | |
| Total applied polymer | 0.5 mg/cm² | |

| | | |
|---|---|---|
| *Quantity based on dry polymer substance [%] | | |

### Capsule Coating Process

The capsules are coated in a fully perforated side-vended pan coating system O'Hara M10. The relevant process parameters are listed in Table 10.

**Table 10: Process Parameter**

| **Parameter** | **Value** |
|---|---|
| Machine | O'Hara M10 |
| Batch size [g] | 864 |
| Nozzle bore [mm] | 1.2 |
| Internal tube diameter [mm] | 2.0 |
| Peristaltic pump | Watson Marlow |
| Atomizing pressure [bar] | 0.8 |
| Flat pattern pressure [bar] | 0.8 |
| Pan speed [rpm] | 10 |
| Inlet air volume [m³/h] | 104 - 116 |
| Inlet air temperature [°C] | 35.0 - 36.1 |
| Inlet air humidity [g/m³] | 4.2 - 7.7 |
| Exhaust air temperature [°C] | 25.2 - 27.0 |
| Exhaust air humidity [g/m³] | 6.1 - 9.6 |
| Product temperature [°C] | 28.1 - 29.6 |
| Spray rate [g/min/kg] | 2.7 - 9.5 |
| Process time [min] | 332 |

### Bridging Test:

The capsules were tested for bridging between body and cap. The test was performed by holding the body and gently twisting the cap of the capsule. If the cap could not be twisted without damaging the capsule, hearing or feeling a cracking and if the cap could not be twisted at all, the capsule failed, and bridging was determined. 100 capsules were tested.

### Results:

Result of this example 1/100 need high forces to sperate capsule cap and body.

### Example 5: (Inventive) Enteric coating of pre-locked capsules in drum coater

The functional and top coat formulations are calculated considering a surface area in pre-locked state of 594.5 mm² and a batch size of 40,000 capsules (K-caps Size 0).

### Functional Coating

Preparations of GMS emulsion, 40% of the water was heated up to 70-80°C. The Polysorbate 80 solution, triethyl citrate and GMS were homogenized in the heated water using a homogenizer (e. g. Ultra Turrax) for 10 minutes. The solids content was approximately 15%. The remaining 60% of water was stirred into the hot GMS emulsion by using a conventional stirrer and cooled down to room temperature while continuous stirring. Then the excipient suspension was poured slowly into the EUDRAGIT^{®} L 30 D-55 dispersion while stirring gently with a conventional stirrer. After 10 minutes gentle stirring the EUDRAGIT^{®} NM 30 D was added slowly under continuous stirring and stirred for further 15 minutes. The final coating suspension was sieved throughout a 300µm sieve and stirred during the coating process. The capsules were coated in the pre-locked state utilizing a drum coater.

**Table 11: Functional Coating**

| **Material** | **Composition** | **Solid Composition Percentage** |
|---|---|---|
| EUDRAGIT^{®} L 30 D-55 | 1.8 mg/cm² | 67.2 % |
| EUDRAGIT^{®} NM 30 D | 0.2 mg/cm² | 7.5% |
| Triethyl citrate | 20.0 % on ds* | 14.9 % |
| Glycerol monostearate | 10.0 % on ds* | 7.5 % |
| Polysorbate 80 | 4.0 % on ds* | 3.0 % |
| Demineralized Water | On demand | n/a |
| | | |
| Solid content | 10.0% w/w | |
| Total solid weight gain | 2.7 mg/cm² | |
| Total applied polymer | 2.0 mg/cm² | |

| | | |
|---|---|---|
| *Quantity based on dry polymer substance [%] | | |

### Top Coating

METHOCEL^{™} VLV was thoroughly dispersed in the water while gently stirring to prevent lumping. 40% of the water was heated up to 70-80°C. The Polysorbate 80 solution, triethyl citrate and GMS were homogenized in the heated water using a homogenizer (e. g. Ultra Turrax) for 10 minutes. The solids content should be approximately 15%. The remaining 60% of water was stirred into the hot GMS emulsion by using a conventional stirrer and cooled down to room temperature while continuous stirring. Pour the suspension slowly into the METHOCEL^{™} VLV solution while stirring gently with a conventional stirrer. Pass the spray suspension through a 0.3 mm sieve. The excipient suspension was added to the polymer dispersion. The spraying suspension was gently stirred during the coating process.

**Table 12: Functional Coating**

| **Material** | **Composition** | **Solid Composition Percentage** |
|---|---|---|
| METHOCEL^{™} VLV | 0.5 mg/cm² | 90.9 % |
| Glycerol monostearate | 5.9 % on ds* | 5.4 % |
| Triethyl citrate | 2.9 % on ds* | 2.6 % |
| Polysorbate 80 | 1.2 % on ds* | 1.1 % |
| Demineralized Water | On demand | n/a |
| | | |
| Solid content | 5% w/w | |
| Total solid weight gain | 0.6 mg/cm² | |

| | | |
|---|---|---|
| *Quantity based on dry polymer substance [%] | | |

### Capsule Coating Process

The capsules are coated in a fully perforated side-vended pan coating system Bohle BFC 40. The relevant process parameters are listed in Table 13.

The equipment parameters were kept equal for functional and top coating.

**Table 13: Process Parameter**

| **Parameter** | **Value** |
|---|---|
| Machine | Bohle BFC 40 |
| Batch size [g] | 4360 |
| Nozzle bore [mm] | 1.0 |
| Atomizing pressure [bar] | 1.0 |
| Flat pattern pressure [bar] | 1.2 |
| Pan speed [rpm] | 8 |
| Inlet air volume [m³/h] | 500 |
| Inlet air temperature [°C] | 38 - 43 |
| Exhaust air temperature [°C] | 30 |
| Product temperature [°C] | 29 - 30 |
| Spray rate [g/min/kg] | 5.7 - 8.0 |
| Process time [min] | 200 |

### Bridging Test:

The capsules were tested for bridging between body and cap. The test was performed by holding the body and gently twisting the cap of the capsule. If the cap could not be twisted without damaging the capsule, hearing or feeling a cracking and if the cap could not be twisted at all, the capsule failed, and bridging was determined. 100 capsules were tested.

### Results:

Result of this example 2/100 need high forces to sperate capsule cap and body.

### Disintegration Test (according to European Pharmacopeia 2.9.1 Test B modified method based on gastro-resistant capsules) - Capsule unfilled

| | |
|---|---|
| Method: | 2h 0.1N HCl followed by full change Buffer system pH 6.8. |
| Apparatus: | PTZ Auto 4 EZ Pharma Test |
| Detection method: | Visually and electrical Impedance |
| Temperature: | 37.0°C |
| Media I: | 700 ml 0.1 N HCL according to European Pharmacopeia |
| Media II: | 700mL pH to 6.8 Phosphate buffer according to European Pharmacopeia |
| Samples: | n=6 |

**Table 14: Disintegration Results**

| Media | Time [min] | Sub Sample 1 n=3 | Sub Sample 2 n=3 |
|---|---|---|---|
| 0.1N HCl | Start | intact | intact |
| 0.1N HCl | 60 minutes | intact | intact |
| 0.1N HCl | 120 minutes | intact | intact |
| pH 6.8 | 5 minutes | disintegrated | disintegrated |

### Dissolution Test (according to European Pharmacopeia (2.9.3) apparatus II)

Capsule manually filled. The polymer coated pre-locked capsules were manually filled with 500mg Caffeine/Lactose Mixture 4:6, closed to the final-locked state and tested in a dissolution test.

### Method:

| | |
|---|---|
| Apparatus: | ERWEKA DT 700 Paddle Apparatus (USP II) |
| Detection method: | Online UV |
| Temperature: | 37.5°C |
| Media I: | 700 ml 0.1 N HCL adjusted to pH 1.2 (by using 2N NaOH and 2N HCl) |
| Media II: | After 2hours in media I 214 ml 0.2 N Na₃PO₄ solution added to increase pH to 6.8 (fine adjustment of pH by using 2N NaOH and 2N HCl) |
| Paddle speed: | 75rpm |

**Table 15: Dissolution Results**

| Media | Time [min] | Sample 1 [% released] | Sample 2 [% released] | Sample 3 [% released] | Sample 4 [% released] | Sample 5 [% released] | Sample 6 [% released] |
|---|---|---|---|---|---|---|---|
| 0.1N HCL | 0 | 0.04 | -0.01 | 0.00 | 0.00 | 0.00 | 0.01 |
| 0.1N HCL | 30 | 0.02 | 0.01 | 0.00 | 0.01 | 0.01 | -0.01 |
| 0.1N HCL | 60 | 0.03 | -0.01 | 0.00 | 0.01 | 0.03 | 0.01 |
| 0.1N HCL | 90 | 0.03 | 0.04 | 0.00 | 0.01 | 0.01 | 0.01 |
| 0.1N HCL | 120 | 0.04 | 0.03 | 0.01 | 0.02 | 0.04 | -0.01 |
| pH6.8 | 125 | 0.24 | 0.21 | 0.32 | 0.24 | 0.33 | 0.18 |
| pH6.8 | 130 | 2.90 | 2.24 | 0.89 | 0.92 | 6.27 | 0.63 |
| pH6.8 | 135 | 87.52 | 25.80 | 62.71 | 63.03 | 60.62 | 94.80 |
| pH6.8 | 140 | 96.36 | 92.35 | 86.98 | 94.08 | 88.04 | 95.53 |
| pH6.8 | 145 | 96.75 | 96.27 | 96.20 | 96.76 | 93.99 | 95.65 |
| pH6.8 | 150 | 96.49 | 95.97 | 96.86 | 96.72 | 95.26 | 95.60 |
| pH6.8 | 165 | 96.76 | 96.41 | 96.69 | 96.92 | 95.62 | 95.73 |
| pH6.8 | 180 | 96.61 | 96.18 | 96.73 | 97.12 | 95.59 | 95.72 |

### Example 6: (Inventive) Enteric coating of pre-locked capsules in drum coater

The functional and top coat formulations are calculated considering a surface area in pre-locked state of 545.82 mm² and a batch size of 9,000 capsules (Capsugel V-Caps Size 0).

### Functional Coating

Preparations of GMS emulsion, 40% of the water was heated up to 70-80°C. The Polysorbate 80 solution, triethyl citrate and GMS were homogenized in the heated water using a homogenizer (e. g. Ultra Turrax) for 10 minutes. The solids content was approximately 15%. The remaining 60% of water was stirred into the hot GMS emulsion by using a conventional stirrer and cooled down to room temperature while continuous stirring. Then the excipient suspension was poured slowly into the EUDRAGIT^{®} L 30 D-55 dispersion while stirring gently with a conventional stirrer. After 10 minutes gentle stirring the EUDRAGIT^{®} FS 30 D was added slowly under continuous stirring and stirred for further 15 minutes. The final coating suspension was sieved throughout a 400µm sieve and stirred during the coating process. The capsules were coated in the pre-locked state utilizing a drum coater.

**Table 16: Functional Coating**

| **Material** | **Composition** | **Solid Composition Percentage** |
|---|---|---|
| EUDRAGIT^{®} L 30 D-55 | 1.75 mg/cm² | 35.7 % |
| EUDRAGIT^{®} FS 30D | 1.75 mg/cm² | 35.7% |
| Triethyl citrate | 20.0 % on ds* | 14.3% |
| Glycerol monostearate | 10.0 % on ds* | 7.1% |
| Polysorbate 80 | 10.0 % on ds* | 7.1% |
| Demineralized Water | On demand | n/a |
| | | |
| Solid content | 10.0% w/w | |
| Total solid weight gain | 4.9 mg/cm² | |
| Total applied polymer | 3.5 mg/cm² | |

| | | |
|---|---|---|
| *Quantity based on dry polymer substance [%] | | |

### Top Coating

METHOCEL^{™} VLV was thoroughly dispersed in the water while gently stirring to prevent lumping. 40% of the water was heated up to 70-80°C. The Polysorbate 80 solution, triethyl citrate and GMS were homogenized in the heated water using a homogenizer (e. g. Ultra Turrax) for 10 minutes. The solids content should be approximately 15%. The remaining 60% of water was stirred into the hot GMS emulsion by using a conventional stirrer and cooled down to room temperature while continuous stirring. Pour the suspension slowly into the METHOCEL^{™} VLV solution while stirring gently with a conventional stirrer. Pass the spray suspension through a 0.3 mm sieve. The excipient suspension was added to the polymer dispersion. The spraying suspension was gently stirred during the coating process.

**Table 17: Top Coating**

| **Material** | **Composition** | **Solid Composition Percentage** |
|---|---|---|
| METHOCEL^{™} VLV | 0.5 mg/cm² | 90.9 % |
| Glycerol monostearate | 5.9 % on ds* | 5.4 % |
| Triethyl citrate | 2.9 % on ds* | 2.6 % |
| Polysorbate 80 | 1.2 % on ds* | 1.1 % |
| Demineralized Water | On demand | n/a |
| | | |
| Solid content | 5% w/w | |
| Total solid weight gain | 0.6 mg/cm² | |
| Total applied polymer | 0.5 mg/cm² | |

| | | |
|---|---|---|
| *Quantity based on dry polymer substance [%] | | |

### Capsule Coating Process

The capsules are coated in a fully perforated side-vended pan coating system O'Hara M10. The relevant process parameters are listed in Table 18.

**Table 18: Process Parameter**

| **Parameter** | **Value** |
|---|---|
| Machine | O'Hara M10 |
| Batch size [g] | 864 |
| Nozzle bore [mm] | 1.2 |
| Internal tube diameter [mm] | 2.0 |
| Peristaltic pump | Watson Marlow |
| Atomizing pressure [bar] | 0.8 |
| Flat pattern pressure [bar] | 0.8 |
| Pan speed [rpm] | 10 |
| Inlet air volume [m³/h] | 105 - 116 |
| Inlet air temperature [°C] | 36.0 - 36.1 |
| Inlet air humidity [g/m³] | 5.0 - 6.1 |
| Exhaust air temperature [°C] | 26.7 - 27.4 |
| Exhaust air humidity [g/m³] | 6.1 - 8.5 |
| Product temperature [°C] | 27.8 - 30.8 |
| Spray rate [g/min/kg] | 3.5 - 8.1 |
| Process time [min] | 382 |

### Bridging Test:

The capsules were tested for bridging between body and cap. The test was performed by holding the body and gently twisting the cap of the capsule. If the cap could not be twisted without damaging the capsule, hearing or feeling a cracking and if the cap could not be twisted at all, the capsule failed, and bridging was determined. 100 capsules were tested.

### Results:

Result of this example 0/100 need high forces to sperate capsule cap and body.

### Example 7: (Inventive) Enteric coating of pre-locked capsules in drum coater

The functional and top coat formulations are calculated considering a surface area in pre-locked state of 464.56 mm² and a batch size of 9,000 capsules (Capsugel V-Caps Size 0).

### Functional Coating

Preparations of GMS emulsion, 40% of the water was heated up to 70-80°C. The Polysorbate 80 solution, triethyl citrate and GMS were homogenized in the heated water using a homogenizer (e. g. Ultra Turrax) for 10 minutes. The solids content was approximately 15%. The remaining 60% of water was stirred into the hot GMS emulsion by using a conventional stirrer and cooled down to room temperature while continuous stirring. Then the excipient suspension was poured slowly into the EUDRAGIT^{®} L 30 D-55 dispersion while stirring gently with a conventional stirrer. After 10 minutes gentle stirring the EUDRAGIT^{®} FS 30 D was added slowly under continuous stirring and stirred for further 15 minutes. The final coating suspension was sieved throughout a 400µm sieve and stirred during the coating process. The capsules were coated in the pre-locked state utilizing a drum coater.

**Table 19: Functional Coating**

| **Material** | **Composition** | **Solid Composition Percentage** |
|---|---|---|
| EUDRAGIT^{®} L 30 D-55 | 1.88 mg/cm² | 35.7 % |
| EUDRAGIT^{®} FS 30D | 1.88 mg/cm² | 35.7% |
| Triethyl citrate | 20.0 % on ds* | 14.3% |
| Glycerol monostearate | 10.0 % on ds* | 7.1% |
| Polysorbate 80 | 10.0 % on ds* | 7.1% |
| Demineralized Water | On demand | n/a |
| | | |
| Solid content | 10.0% w/w | |
| Total solid weight gain | 5.25 mg/cm² | |
| Total applied polymer | 3.75 mg/cm² | |

| | | |
|---|---|---|
| *Quantity based on dry polymer substance [%] | | |

### Top Coating

METHOCEL^{™} VLV was thoroughly dispersed in the water while gently stirring to prevent lumping. 40% of the water was heated up to 70-80°C. The Polysorbate 80 solution, triethyl citrate and GMS were homogenized in the heated water using a homogenizer (e. g. Ultra Turrax) for 10 minutes. The solids content should be approximately 15%. The remaining 60% of water was stirred into the hot GMS emulsion by using a conventional stirrer and cooled down to room temperature while continuous stirring. Pour the suspension slowly into the METHOCEL^{™} VLV solution while stirring gently with a conventional stirrer. Pass the spray suspension through a 0.3 mm sieve. The excipient suspension was added to the polymer dispersion. The spraying suspension was gently stirred during the coating process.

**Table 20: Top Coating**

| **Material** | **Composition** | **Solid Composition Percentage** |
|---|---|---|
| METHOCEL^{™} VLV | 0.25 mg/cm² | 90.9 % |
| Glycerol monostearate | 5.9 % on ds* | 5.4 % |
| Triethyl citrate | 2.9 % on ds* | 2.6 % |
| Polysorbate 80 | 1.2 % on ds* | 1.1 % |
| Demineralized Water | On demand | n/a |
| | | |
| Solid content | 5% w/w | |
| Total solid weight gain | 0.28 mg/cm² | |
| Total applied polymer | 0.25 mg/cm² | |

| | | |
|---|---|---|
| *Quantity based on dry polymer substance [%] | | |

### Capsule Coating Process

The capsules are coated in a fully perforated side-vended pan coating system O'Hara M10. The relevant process parameters are listed in Table 21Table.

**Table 21: Process Parameter**

| **Parameter** | **Value** |
|---|---|
| Machine | O'Hara M10 |
| Batch size [g] | 684 |
| Nozzle bore [mm] | 1.2 |
| Internal tube diameter [mm] | 2.0 |
| Peristaltic pump | Watson Marlow |
| Atomizing pressure [bar] | 0.8 |
| Flat pattern pressure [bar] | 0.8 |
| Pan speed [rpm] | 10 |
| Inlet air volume [m³/h] | 110-118 |
| Inlet air temperature [°C] | 35.0 - 37.1 |
| Inlet air humidity [g/m³] | 5.3 - 6.5 |
| Exhaust air temperature [°C] | 25.0 - 28.2 |
| Exhaust air humidity [g/m³] | 6.6 - 9.6 |
| Product temperature [°C] | 25.4 - 29.0 |
| Spray rate [g/min/kg] | 4.0 - 8.4 |
| Process time [min] | 314 |

### Bridging Test:

The capsules were tested for bridging between body and cap. The test was performed by holding the body and gently twisting the cap of the capsule. If the cap could not be twisted without damaging the capsule, hearing or feeling a cracking and if the cap could not be twisted at all, the capsule failed, and bridging was determined. 100 capsules were tested.

### Results:

Result of this example 0/100 need high forces to sperate capsule cap and body.

### Example 8: (Inventive) Enteric coating of pre-locked capsules in drum coater

The functional and top coat formulations are calculated considering a surface area in pre-locked state of 464.56 mm² and a batch size of 9,000 capsules (Capsugel V-Caps Size 0).

### Functional Coating

Preparations of GMS emulsion, 40% of the water was heated up to 70-80°C. The Polysorbate 80 solution, triethyl citrate and GMS were homogenized in the heated water using a homogenizer (e. g. Ultra Turrax) for 10 minutes. The solids content was approximately 15%. The remaining 60% of water was stirred into the hot GMS emulsion by using a conventional stirrer and cooled down to room temperature while continuous stirring. Then the excipient suspension was poured slowly into the EUDRAGIT^{®} L 30 D-55 dispersion while stirring gently with a conventional stirrer. After 10 minutes gentle stirring the EUDRAGIT^{®} NM 30 D was added slowly under continuous stirring and stirred for further 15 minutes. The final coating suspension was sieved throughout a 400µm sieve and stirred during the coating process. The capsules were coated in the pre-locked state utilizing a drum coater.

**Table 22: Functional Coating**

| **Material** | **Composition** | **Solid Composition Percentage** |
|---|---|---|
| EUDRAGIT^{®} L 30 D-55 | 2.63 mg/cm² | 50.0% |
| EUDRAGIT^{®} FS 30D | 1.13 mg/cm² | 21.4% |
| Triethyl citrate | 20.0 % on ds* | 14.3% |
| Glycerol monostearate | 10.0 % on ds* | 7.1% |
| Polysorbate 80 | 10.0 % on ds* | 7.1% |
| Demineralized Water | On demand | n/a |
| | | |
| Solid content | 10.0% w/w | |
| Total solid weight gain | 5.25 mg/cm² | |
| Total applied polymer | 3.76 mg/cm² | |

| | | |
|---|---|---|
| *Quantity based on dry polymer substance [%] | | |

### Top Coating

METHOCEL^{™} VLV was thoroughly dispersed in the water while gently stirring to prevent lumping. 40% of the water was heated up to 70-80°C. The Polysorbate 80 solution, triethyl citrate and GMS were homogenized in the heated water using a homogenizer (e. g. Ultra Turrax) for 10 minutes. The solids content should be approximately 15%. The remaining 60% of water was stirred into the hot GMS emulsion by using a conventional stirrer and cooled down to room temperature while continuous stirring. Pour the suspension slowly into the METHOCEL^{™} VLV solution while stirring gently with a conventional stirrer. Pass the spray suspension through a 0.3 mm sieve. The excipient suspension was added to the polymer dispersion. The spraying suspension was gently stirred during the coating process.

**Table 23: Top Coating**

| **Material** | **Composition** | **Solid Composition Percentage** |
|---|---|---|
| METHOCEL^{™} VLV | 0.25 mg/cm² | 90.9 % |
| Glycerol monostearate | 5.9 % on ds* | 5.4 % |
| Triethyl citrate | 2.9 % on ds* | 2.6 % |
| Polysorbate 80 | 1.2 % on ds* | 1.1 % |
| Demineralized Water | On demand | n/a |
| | | |
| Solid content | 5% w/w | |
| Total solid weight gain | 0.28 mg/cm² | |
| Total applied polymer | 0.25 mg/cm² | |

| | | |
|---|---|---|
| *Quantity based on dry polymer substance [%] | | |

### Capsule Coating Process

The capsules are coated in a fully perforated side-vended pan coating system O'Hara M10. The relevant process parameters are listed in Table 24.

**Table 24: Process Parameter**

| **Parameter** | **Value** |
|---|---|
| Machine | O'Hara M10 |
| Batch size [g] | 684 |
| Nozzle bore [mm] | 1.2 |
| Internal tube diameter [mm] | 2.0 |
| Peristaltic pump | Watson Marlow |
| Atomizing pressure [bar] | 0.8 |
| Flat pattern pressure [bar] | 0.8 |
| Pan speed [rpm] | 10 |
| Inlet air volume [m³/h] | 112 - 122 |
| Inlet air temperature [°C] | 35.0 - 38.0 |
| Inlet air humidity [g/m³] | 5.4 - 6.2 |
| Exhaust air temperature [°C] | 25.9 - 28.8 |
| Exhaust air humidity [g/m³] | 6.6 - 8.9 |
| Product temperature [°C] | 26.6 - 29.2 |
| Spray rate [g/min/kg] | 4.1 - 8.6 |
| Process time [min] | 324 |

### Bridging Test:

The capsules were tested for bridging between body and cap. The test was performed by holding the body and gently twisting the cap of the capsule. If the cap could not be twisted without damaging the capsule, hearing or feeling a cracking and if the cap could not be twisted at all, the capsule failed, and bridging was determined. 100 capsules were tested.

### Results:

Result of this example 2/100 need high forces to sperate capsule cap and body.

### Example 9: (Inventive) Filling of enteric coated capsules with RNA-containing lipid nanoparticles and pH dependent release of lipid nanoparticles

In this example FLuc mRNA containing lipid nanoparticles (LNPs) are applied as a relevant model drug product for combination with enteric coated capsules.

### Preparation of LNPs

**Table 25: Lipids for LNP preparation**

| Lipid | Provider | Catalogue Number |
|---|---|---|
| 1,2-dioleyloxy-3-dimethylaminopropane (DODMA) | NOF America Corporation | COATSOME^{®} CL-E8181DA |
| Cholesterol | Sigma-Aldrich | C1231 |
| 1,2-distearoyl-sn-glycero-3-phosphocholine (DSPC) | NOF America Corporation | COATSOME^{®} MC-8080 |
| 1,2-Dimyristoyl-rac-glycero-3-methoxypolyethylene glycol-2000 (PEG2K-DMG) | Sigma-Aldrich | 880151P |

1.4 mL of an ethanolic solution containing 9.44 mM total lipid (50 mol% DODMA, 38 mol% cholesterol, 10 mol% DSPC, 2 mol% PEG2K-DMG) were mixed with 4.2 mL of an RNAse free aqueous solution containing 0.133 g/L CleanCap^{®} FLuc mRNA (TriLink, L-7602) and 11.01 mM acetic acid using the Nanoassemblr Benchtop (PNI) platform. The crude LNP solution was dialyzed (Slide-A-Lyzer^{™}, 10K MWCO) against 10 mM HEPES pH 7 buffer for 3 hours (3x buffer exchange). After dialysis, RNAse free sucrose solution (20 wt%) was added to the LNP solution to achieve a final sucrose concentration of 10 wt%. LNPs were lyophilized over 48 hours and stored at 4 °C until further use.

### Capsule filling and dissolution assay

Lyophilized LNPs were filled into enteric coated capsules of examples 5 & 8 at an amount equal to 100 µg of mRNA per capsule. The filled capsules were sealed and stored at 4 °C until further use. To simulate the gastric environment in fed state the capsules were incubated on a rocking shaker for 2 hours at 37 °C in 10 mL of 0.1 N HCl containing 2 g/L pepsin. Samples for release analysis were taken after 60 and 120 minutes. Subsequently, acidic medium was exchanged against 10 mL of 0.2 M phosphate buffer pH 6.8 and capsules were incubated for another 60 minutes with sample-taking in 15 minutes intervals.

As a negative control pure LNPs without capsule protection were incubated under the same conditions: 13.9 µL of LNP solution (containing 36 ng/µL mRNA) were mixed with 50 µL 0.1 N HCl containing 2 g/L pepsin and incubated for 2 hours on an orbital shaker at 37 °C and 300 rpm. Afterwards, 36.1 µL of phosphate buffer were added to the mixture and incubation was continued for another 60 minutes.

After the dissolution assay the media containing the dissolved capsules and LNPs were immediately used for the cell transfection assay without intermediate storage. The samples taken at fixed time intervals were stored at 4 °C until further analysis in Ribogreen assay.

### Ribogreen assay to assess LNP/capsule release kinetics

Ribogreen assay was applied in order to detect and quantify RNA after release of LNPs from capsules. mRNA concentration was measured at different time intervals to establish release kinetics. Before staining of mRNA with Ribogreen dye LNPs were either treated with Triton X-100 or left untreated. This allows measurement of total mRNA (after breaking of particle structure with Triton X-100) or measurement of accessible mRNA only, within intact particles.

### Experimental procedure

The Quant-iT^{™} RiboGreen^{™} RNA Assay Kit was used for this assay. As Ribogreen assay is based on measuring fluorescence, black 96-well assay plates with a clear bottom were applied. The procedure was performed according to manufacturer's protocol with slight adjustments. In a first step, 1x TE buffer was prepared by dilution of buffer stock with RNAse free water. A 2% Triton buffer was prepared by mixing 1 ml Triton X-100 with 50 ml 1x TE buffer and subsequent stirring for 15 minutes. LNP samples were diluted to a theoretical concentration of 1 µg/ml using TE buffer and added to the plate at a volume of 50 µl. Either 50 µl of Triton buffer or of TE buffer were added to the samples in order to measure total mRNA or accessible mRNA only. For dissolution of LNPs in the presence of Triton-buffer the plate was placed into an incubator for 10 minutes at 37 °C and 5% CO2. A calibration standard with the corresponding Fluc mRNA and buffers was applied and added to the same plate as the samples. Working solution of Ribogreen dye was prepared by a 1:100 dilution of reagent with TE-buffer. 100 µl working solution were added to each well followed by thorough mixing through pipetting up and down. Fluorescence signals were measured with a microplate reader at an excitation / emission value of 480 / 520 nm. All samples and standards were measured in duplicates.

### Results

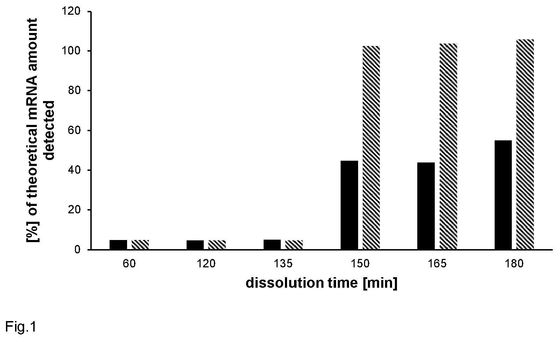

The results are shown in Fig. 1. Fig. 1 shows release kinetics of mRNA-LNPs from capsules of examples 5 and 8 after dissolution assay in 0.1 N HCl (0-120 minutes) and phosphate buffer pH 6.8 (120-180 minutes) as obtained by Ribogreen assay (representative from n=3). Dark black bars: intact LNPs, shaded bars: LNPs dissolved with Triton X-100.

The Ribogreen Assay clearly proofs a pH dependent release of mRNA-LNPs out of the enteric coated capsules. Both, measurement of total mRNA as well as of accessible mRNA within LNPs give the same release kinetics.

Within 30 minutes after exchange of incubation medium from acidic to pH 6.8 LNPs were fully rehydrated and released from the capsules which went along with complete capsule dissolution. Importantly, no release of LNPs and mRNA was observed during the 120 minutes incubation in 0.1 N HCl which confirms the structural integrity of the capsules under acidic conditions.

### Transfection assay to assess LNP activity before and after capsule filling and release

Luciferase transfection assay in human epithelial cells (HeLa) cells was applied in order to assess LNP functionality after release from capsules of example 5 and 8. Lyophilized LNPs which were rehydrated only or additionally incubated in fed state simulated gastric and intestinal fluids without capsule protection served as positive and negative controls, respectively.

### Experimental procedure

One day before transfection 10,000 cells per well were seeded into a 96-well plate and cultured for 24 h at 37 °C and 5% CO₂. On day 2, old medium was removed and 90 µL of fresh medium was added to the cells. All samples were adjusted to an mRNA concentration of 5 ng/µL using RNAse free water for dilution. 10 µL of the respective diluted samples were added to the cells equaling an amount of 50 ng mRNA per well in a total volume of 100 µL. The cells were further incubated for 24 h at 37 °C and 5% CO₂. On day 3, transfection efficiency was determined using a luciferase kit system according to manufacturer's protocol (Promega GmbH). By adding a luciferase substrate to the cells, a luminescence signal is generated which can be quantified by a multiplate reader (Plate reader Infinite^{®} 200 PRO, Tecan).

### Results

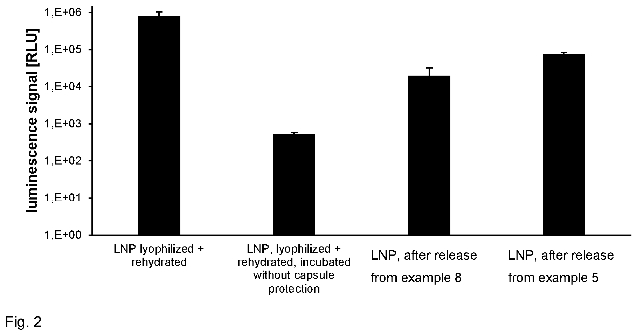

The results are shown in Fig. 2. Fig. 2 shows the transfection efficiency of LNP samples after different pre-treatments. 50 ng of mRNA per well were applied for each condition.

The luciferase assay demonstrates the functionality of the LNPs after release from capsules as HeLa cells incubated with these samples showed distinct expression of the embedded Fluc mRNA. Protection of the LNPs against fed state simulated gastric and intestinal fluids is further verified by considering the LNP negative control which was exposed to the same media without any capsule protection. Transfection efficiency of capsule protected LNPs is 1.5 to 2 logs higher than efficiency of non-protected LPNs confirming a clear beneficial effect of enteric coated capsules on LNP functionality.

Compared to the positive control, i.e. lyophilized LNPs rehydrated and directly applied for transfection assay, efficiency of released LNPs is ~1 log lower. This can be attributed to dissolved capsule ingredients which might interact with LNPs and compromise particle integrity. Importantly, capsules of example 5 show a ~0.5 log better performance than those of example 8 indicating a better compatibility with LNPs, possibly due to differences in capsule composition.

### Example 10: (Comparative) Enteric coating of pre-locked capsules in drum coater

The functional coat formulation is calculated considering a surface area in pre-locked state of 545.82 mm² and a batch size of 3,125 capsules (Capsugel VcapsPlus Size 0).

### Functional Coating

Preparations of GMS emulsion, 40% of the water was heated up to 70-80°C. The Polysorbate 80 solution, triethyl citrate and GMS were homogenized in the heated water using a homogenizer (e. g. Ultra Turrax) for 10 minutes. The solids content was approximately 15%. The remaining 60% of water was stirred into the hot GMS emulsion by using a conventional stirrer and cooled down to room temperature while continuous stirring. Then the excipient suspension was poured slowly into the EUDRAGIT^{®} L 30 D-55 dispersion while stirring gently with a conventional stirrer and stirred for further 15 minutes. The final coating suspension was sieved throughout a 300µm sieve and stirred during the coating process. The capsules were coated in the pre-locked state utilizing a drum coater.

**Table 26: Functional Coating**

| **Material** | **Composition** | **Solid Composition Percentage** |
|---|---|---|
| EUDRAGIT^{®} L 30 D-55 | 4 mg/cm² | 77.8 % |
| Triethyl citrate | 18.1 % on ds* | 14.1 % |
| Glycerol monostearate | 7.5 % on ds* | 5.8 % |
| Polysorbate 80 | 3.0 % on ds* | 2.3 % |
| Demineralized Water | On demand | n/a |
| | | |
| Solid content | 16.0% w/w | |
| Total solid weight gain | 5.1 mg/cm² | |
| Total applied polymer | 4.0 mg/cm² | |

| | | |
|---|---|---|
| *Quantity based on dry polymer substance [%] | | |

### Capsule Coating Process

The capsules are coated in a fully perforated side-vended pan coating system Lödinge LHC 15/30/36. The relevant process parameters are listed in Table 27.

**Table 27: Process Parameter**

| **Parameter** | **Value** |
|---|---|
| Machine | Lödige LHC |
| Batch size [g] | 300 |
| Nozzle bore [mm] | 1.0 |
| Atomizing pressure [bar] | 0.5 |
| Flat pattern pressure [bar] | 0.5 |
| Pan speed [rpm] | 15 |
| Inlet air volume [m³/h] | 90 - 92 |
| Inlet air temperature [°C] | 37.5 - 40 |
| Exhaust air temperature [°C] | 24.7 - 32.0 |
| Inlet air humidity [% r.h.] | 22.6 - 23.1 |
| Exhaust air humidity [%r.h.] | 37.1 - 47.0 |
| Spray rate [g/min/kg] | 11.7 - 35 |
| Process time [min] | 75 |

In process samples have been taken after 2 mg/cm² and 3 mg/cm² polymer weight gain

### Bridging Test:

The capsules were tested for bridging between body and cap. The test was performed by holding the body and gently twisting the cap of the capsule. If the cap could not be twisted without damaging the capsule, hearing or feeling a cracking and if the cap could not be twisted at all, the capsule failed, and bridging was determined. 100 capsules were tested.

### Results:

Result of this example 0/100 need high forces to sperate capsule cap and body.

### Encapsulation Parameter

A 400 mg of a 50:50 blend with MCC and Caffeine was filled into the polymer coated pre-locked capsules using an automatic MG2 Labby Capsule filling equipment with a powder filling set up using standard format size 0 tooling for capsule opening, transport, filling and closing. The machine output was set to 2000 cps/hour.

Capsules tested in automatic capsule filling machine, 2.6 and 3.9 mg/cm² total solid weight gain feasible to process automatically. At 5.1 mg/cm² total solid weight gain the limitation was the standard tooling which was not able to operate with the pre-locked capsules due to the increased layer thickness. In order to investigate, if polymer weight gains above 4 mg/cm² for that particular formulation could observed modified tooling would be required considering the increased capsule diameter.

### Dissolution Test (according to European Pharmacopeia (2.9.3) apparatus II)

### Method:

| | |
|---|---|
| Apparatus: | ERWEKA DT 700 Paddle Apparatus (USP II) |
| Detection method: | Online UV |
| Temperature: | 37.5°C |
| Media I: | 700 ml 0.1 N HCL adjusted to pH 1.2 (by using 2N NaOH and 2N HCl) |
| Media II: | After 2hours in media I 214 ml 0.2 N Na₃PO₄ solution added to increase pH to 6.8 (fine adjustment of pH by using 2N NaOH and 2N HCl) |
| Paddle speed: | 75rpm |
| Samples: | Vessel 1 - 3 in-process sample with 2 mg/cm²weight gain |
| | Vessel 4 - 6 in-process sample with 3 mg/cm²weight gain |

**Table 28: Dissolution Results**

| Media | Time [min] | Vessel 1 [% released] | Vessel 2 [% released] | Vessel 3 [% released] | Vessel 4 [% released] | Vessel 5 [% released] | Vessel 6 [% released] |
|---|---|---|---|---|---|---|---|
| 0.1N HCL | 0 | 0.00 | 0.00 | -0.01 | -0.01 | -0.01 | -0.01 |
| 0.1N HCL | 30 | 0.06 | 0.03 | -0.01 | 0.09 | 0.11 | 0.04 |
| 0.1N HCL | 60 | 0.05 | 0.08 | 0.04 | 0.11 | 0.15 | 0.05 |
| 0.1N HCL | 90 | 0.07 | 0.10 | 0.05 | 0.12 | 0.17 | 0.07 |
| pH6.8 | 120 | 0.13 | 0.12 | 0.06 | 0.11 | 0.17 | 0.05 |
| pH6.8 | 125 | 0.24 | 0.26 | 0.17 | 0.19 | 0.30 | 0.16 |
| pH6.8 | 130 | 99.03 | 89.32 | 74.29 | 17.15 | 49.25 | 29.63 |
| pH6.8 | 135 | 102.93 | 101.09 | 97.93 | 98.22 | 78.60 | 89.12 |
| pH6.8 | 140 | 103.31 | 101.64 | 99.87 | 100.91 | 94.52 | 98.70 |
| pH6.8 | 150 | 103.46 | 101.61 | 101.90 | 101.06 | 100.10 | 100.05 |
| pH6.8 | 165 | 103.56 | 101.69 | 101.93 | 101.21 | 100.55 | 100.03 |
| pH6.8 | 180 | 103.90 | 101.71 | 102.11 | 101.20 | 100.18 | 100.06 |

### Influx Detection via Dissolution Test (according to European Pharmacopeia (2.9.3) apparatus II)

### Method:

| | |
|---|---|
| Apparatus: | ERWEKA DT 700 Paddle Apparatus (USP II) |
| Detection method: | Online UV |
| Temperature: | 37.5°C |
| Media I: | 700 ml 0.1 N HCL adjusted to pH 1.2 (by using 2N NaOH and 2N HCl) |
| Media II: | After 2hours in media I 214 ml 0.2 N Na₃PO₄ solution added to increase pH to 6.8 (fine adjustment of pH by using 2N NaOH and 2N HCl) |
| Paddle speed: | 50rpm |
| Samples: | Vessel 1 - 3 in-process sample with 2 mg/cm²weight gain |
| | Vessel 4 - 6 in-process sample with 3.5 mg/cm²weight gain |
| | Vessel 7 - 9 in-process sample with 4.0 mg/cm²weight gain |
| | Vessel 10 - 12 in-process sample with 5.0 mg/cm²weight gain |

Samples have been filled manually with Omeprazole in order to detect acid uptake. Omeprazole changes color to reddish/ brownish upon contact to acid due to a chemical degradation.

### Results:

| | |
|---|---|
| Vessel 1 - 3 | strong color change in all three vessels |
| Vessel 4 - 6 | strong color change in one vessel, minimal color change in two vessel |
| Vessel 7 - 9 | strong color change in one vessel, minimal color change in two vessel |
| Vessel 10 - 12 | moderate color change in one vessel, medium color change in two vessel |

### Example 11: (Comparative) Enteric coating of pre-locked capsules in drum coater

The functional coat formulation is calculated considering a surface area in pre-locked state of 545.82 mm² and a batch size of 3,125 capsules (Capsugel VcapsPlus Size 0).

### Functional Coating

Preparations of GMS emulsion, 40% of the water was heated up to 70-80°C. The Polysorbate 80 solution, triethyl citrate and GMS were homogenized in the heated water using a homogenizer (e. g. Ultra Turrax) for 10 minutes. The solids content was approximately 15%. The remaining 60% of water was stirred into the hot GMS emulsion by using a conventional stirrer and cooled down to room temperature while continuous stirring. Then the excipient suspension was poured slowly into the EUDRAGIT^{®} L 30 D-55 dispersion while stirring gently with a conventional stirrer. After 10 minutes gentle stirring the EUDRAGIT^{®} FS 30 D was added slowly under continuous stirring and stirred for further 15 minutes. The final coating suspension was sieved throughout a 300µm sieve and stirred during the coating process. The capsules were coated in the pre-locked state utilizing a drum coater.

**Table 29: Functional Coating**

| **Material** | **Composition** | **Solid Composition Percentage** |
|---|---|---|
| EUDRAGIT^{®} L 30 D-55 | 1.2 mg/cm² | 24.0 % |
| EUDRAGIT^{®} FS 30 D | 4.8 mg/cm² | 56.0 % |
| Triethyl citrate | 18.1 % on ds* | 5.8 % |
| Glycerol monostearate | 7.5 % on ds* | 5.8 % |
| Polysorbate 80 | 3.0 % on ds* | 8.5 % |
| Demineralized Water | On demand | n/a |
| | | |
| Solid content | 16.0% w/w | |
| Total solid weight gain | 5.1 mg/cm² | |
| Total applied polymer | 4.0 mg/cm² | |

| | | |
|---|---|---|
| *Quantity based on dry polymer substance [%] | | |

### Capsule Coating Process

The capsules are coated in a fully perforated side-vended pan coating system Lödige LHC 15/30/36. The relevant process parameters are listed in Table 30.

**Table 30: Process Parameter**

| **Parameter** | **Value** |
|---|---|
| Machine | Lödige LHC |
| Batch size [g] | 300 |
| Nozzle bore [mm] | 1.0 |
| Atomizing pressure [bar] | 0.6 |
| Flat pattern pressure [bar] | 0.6 |
| Pan speed [rpm] | 15 |
| Inlet air volume [m³/h] | 94 |
| Inlet air temperature [°C] | 35.5 - 38.7 |
| Exhaust air temperature [°C] | 25.1 - 27.4 |
| Inlet air humidity [% r.h.] | 44.1 - 45.5 |
| Exhaust air humidity [%r.h.] | 37.3 - 43.8 |
| Spray rate [g/min/kg] | 10.7 - 23.3 |
| Process time [min] | 75 |

In process samples have been taken after 1 mg/cm², 2 mg/cm² and 3 mg/cm² polymer weight gain

### Bridging Test:

The capsules were tested for bridging between body and cap. The test was performed by holding the body and gently twisting the cap of the capsule. If the cap could not be twisted without damaging the capsule, hearing or feeling a cracking and if the cap could not be twisted at all, the capsule failed, and bridging was determined. 100 capsules were tested.

### Results:

Result of this example 0/100 need high forces to sperate capsule cap and body.

### Encapsulation Parameter

A 400 mg of a 50:50 blend with MCC and Caffeine was filled into the polymer coated pre-locked capsules using an automatic MG2 Labby Capsule filling equipment with a powder filling set up using standard format size 0 tooling for capsule opening, transport, filling and closing. The machine output was set to 2000 cps/hour.

Capsules tested in automatic capsule filling machine, 1.25 mg/cm² total solid weight gain feasible to process automatically. At 2.5 mg/cm² and higher total solid weight gain the limitation was the flowability of the capsules into standard tooling which was not able to operate with the pre-locked capsules due to the increased layer thickness and stickiness of the pre-coated capsules.

### Dissolution Test (according to European Pharmacopeia (2.9.3) apparatus II)

### Method:

| | |
|---|---|
| Apparatus: | ERWEKA DT 700 Paddle Apparatus (USP II) |
| Detection method: | Online UV |
| Temperature: | 37.5°C |
| Media I: | 700 ml 0.1 N HCL adjusted to pH 1.2 (by using 2N NaOH and 2N HCl) |
| Media II: | After 2hours in media I 214 ml 0.2 N Na3PO4 solution added to increase pH to 6.8 (fine adjustment of pH by using 2N NaOH and 2N HCl) |
| Paddle speed: | 75rpm |
| Samples: | Vessel 1 - 3 in-process sample with 1.25 mg/cm²weight gain |

**Table 31: Dissolution Results**

| Media | Time [min] | Vessel 1 [% released] | Vessel 2 [% released] | Vessel 3 [% released] |
|---|---|---|---|---|
| 0.1N HCL | 0 | 0.01 | 0.01 | 0.01 |
| 0.1N HCL | 60 | 9.90 | 0.79 | 15.50 |
| 0.1N HCL | 120 | 96.68 | 5.51 | 73.74 |
| pH6.8 | 150 | 102.36 | 40.04 | 104.21 |
| pH6.8 | 180 | 103.95 | 103.28 | 104.41 |
| pH6.8 | 190 | 105.27 | 105.44 | 104.73 |
| pH6.8 | 200 | 105.60 | 105.54 | 104.48 |
| pH6.8 | 210 | 105.40 | 105.58 | 104.51 |
| pH6.8 | 225 | 105.41 | 105.63 | 104.54 |
| pH6.8 | 240 | 105.64 | 105.65 | 104.54 |
| pH6.8 | 300 | 105.70 | 106.08 | 104.62 |
| pH6.8 | 0 | 0.01 | 0.01 | 0.01 |

### Example 12: (Comparative) Enteric coating of pre-locked capsules in drum coater

The functional and top coat formulations are calculated considering a surface area in pre-locked state of 594.5 mm² and a batch size of 5,000 capsules (K-caps Size 0).

### Functional Coating

Triethyl citrate and water were poured slowly into the EUDRAGIT^{®} L 30 D-55 dispersion while stirring gently with a conventional stirrer. After 10 minutes gentle stirring the EUDRAGIT^{®} NM 30 D was added slowly under continuous stirring and stirred for further 15 minutes. The final coating suspension was sieved throughout a 300µm sieve and stirred during the coating process. The capsules were coated in the pre-locked state utilizing a drum coater.

**Table 32: Functional Coating**

| **Material** | **Composition** | **Solid Composition Percentage** |
|---|---|---|
| EUDRAGIT^{®} L 30 D-55 | 1.8 mg/cm² | 81.8 % |
| EUDRAGIT^{®} NM 30 D | 0.2 mg/cm² | 9.1 % |
| Triethyl citrate | 20.0 % on ds* | 9.1 % |
| Demineralized Water | On demand | n/a |
| | | |
| Solid content | 10.0% w/w | |
| Total solid weight gain | 2.2 mg/cm² | |
| Total applied polymer | 2.0 mg/cm² | |

| | | |
|---|---|---|
| *Quantity based on dry polymer substance [%] | | |

### Top Coating

METHOCEL^{™} E3 was thoroughly dispersed in the water while gently stirring to prevent lumping until the solids are completely dissolved. Stir for further 15 minutes and pass the spray suspension through a 0.3 mm sieve. The excipient suspension was added to the polymer dispersion. The spraying suspension was gently stirred during the coating process.

**Table 33: Functional Coating**

| **Material** | **Composition** | **Solid Composition Percentage** |
|---|---|---|
| METHOCEL^{™} E3 | 0.5 mg/cm² | 100 % |
| Demineralized Water | On demand | n/a |
| | | |
| Solid content | 5% w/w | |
| Total solid weight gain | 0.5 mg/cm² | |

| | | |
|---|---|---|
| *Quantity based on dry polymer substance [%] | | |

### Capsule Coating Process

The capsules were coated using a Neocota, 14 inch perforated drum coating system.

### Bridging Test:

The capsules were tested for bridging between body and cap. The test was performed by holding the body and gently twisting the cap of the capsule. If the cap could not be twisted without damaging the capsule, hearing or feeling a cracking and if the cap could not be twisted at all, the capsule failed, and bridging was determined. 100 capsules were tested.

### Encapsulation Parameter

A 472 mg of a 60:40 blend with MCC, and Caffeine was filled into the polymer coated pre-locked capsules using an automatic Bosch GKF 400 capsule filling equipment with a powder filling set up using standard format size 0 tooling for capsule opening, transport, filling and closing. The machine output was set to 12,000 cps/hour.

### Disintegration Test (according to European Pharmacopeia 2.9.1 Test B modified method based on gastro-resistant capsules)

| | |
|---|---|
| Method: | 2h 0.1N HCl followed by full change Buffer system pH 6.8. |
| Apparatus: | PTZ Auto 4 EZ Pharma Test |
| Detection method: | Visually and electrical Impedance |
| Temperature: | 37.0°C |
| Media I: | 700 ml 0.1 N HCL according to European Pharmacopeia |
| Media II: | 700 mL pH to 6.8 Phosphate buffer according to European Pharmacopeia |
| Samples: | n=6 |

**Table 34: Disintegration Results**

| Media | Time [min] | Sub Sample 1 n=3 | Sub Sample 2 n=3 |
|---|---|---|---|
| 0.1N HCl | Start | intact | intact |
| 0.1N HCl | 60 minutes | intact | intact |
| 0.1N HCl | 120 minutes | intact | intact |
| pH 6.8 | 30 minutes | disintegrated | disintegrated |

### Moisture uptake during disintegration testing

Additional disintegrations tests were performed according to the method described above. An n = 6 investigation per formulations have been performed and stopped after 2 hours in 0.1N HCl. The capsule weight was determined before and after media exposure in order to evaluate the media uptake by the capsule. Capsules have been filled with a powder test composition of total weight of 500 mg lactose. Capsules were gently dried with a wipe before weighing.

**Table 35: Media uptake during disintegration testing**

| Formulations | Example 12 | | |
|---|---|---|---|
| Ratio 1^{st} / 2^{nd} layer | 82/18 | 87/13 | 90/10 |
| Total solid weight gain (total coating amount) | 2.7mg/cm² | 3.8 mg/cm² | 4.9 mg/cm² |
| Average media uptake | 6.29 % | 6.06 % | 5.32% |

### Conclusion:

Successful coating with no bridged capsules. Capsules tested in automatic capsule filling machine, 2.2 mg/cm² total solid weight gain feasible to process automatically. Disintegration resistance for 2 hours in 0.1N HCL and disintegration after 30 minutes in phosphate buffer pH 6.8.

Media uptake of inventive examples 2 - 8 has been determined after exposure in 2 hours 0.1 N HCl at 0.03 to 2.31 which was significant less than for comparative example 12. Especially example 5 and 7 showed significantly low levels of uptake at 0.05 % and 0.26 %, respectively. Those capsule compositions were successfully tested in example 9 with highly acid sensitive FLuc mRNA.

## Claims

1. Process for preparing a polymer-coated hard shell capsule comprising at least a functional coat and a top coat, suitable as container for pharmaceutical or nutraceutical biologically active ingredients, wherein the hard shell capsule comprises a body and a cap, wherein in the closed state the cap overlaps the body either in a pre-locked state or in a final-locked state, wherein the hard shell capsule is provided in the pre-locked state and
is coated
with a first coating solution, suspension or dispersion comprising or consisting of
a1) at least one polymer;
b1) optionally at least one glidant;
c1) optionally at least one emulsifier;
d1) optionally at least one plasticizer;
e1) optionally at least one biologically active ingredient; and
f1) optionally at least one additive, different from a1) to e1);
to obtain the functional coat of the hard shell capsule in the pre-locked state; and thereafter is coated with a second coating solution, suspension or dispersion, which is different from the first coating solution, suspension or dispersion, comprising or consisting of
a2) at least one polymer;
b2) optionally at least one glidant;
c2) at least one emulsifier;
d2) at least one plasticizer;
e2) optionally at least one biologically active ingredient; and
f2) optionally at least one additive, different from a2) to e2);
to obtain the top coat of the hard shell capsule in the pre-locked state, wherein
the total coating amount is 2.0 to 10 mg/cm²; and
the coating amount of the top coat is at most 40 % of the coating amount of the functional coat.

2. Process according to claim 1, wherein the base material of the body and the cap is selected from hydroxypropyl methyl cellulose, starch, gelatin, pullulan and a copolymer of a C1- to C4-alkylester of (meth)acrylic acid and (meth)acrylic acid.

3. Process according to claim 1 or 2, wherein the at least one polymer a1) and/or a2 is selected from at least one (meth)acrylate copolymer.

4. Process according to claim 1 or 2, wherein the at least one polymer a1) and/or a2) is
i) a Core-Shell polymer, which is a copolymer obtained by a two stage emulsion polymerization process with a core with 70 to 80 % by weight, comprising polymerized units of 65 to 75 % by weight of ethyl acrylate and 25 to 35 % by weight of methyl methacrylate, and a shell with 20 to 30 % by weight, comprising polymerized units of 45 to 55 % by weight ethyl acrylate and 45 to 55 % by weight methacrylic acid; or
ii) an anionic polymer obtained by polymerizing 25 to 95 % by weight C1- to C12-alkyl esters of acrylic acid or of methacrylic acid and 75 to 5% by weight (meth)acrylate monomers with an anionic group; or
iii) a cationic (meth)acrylate copolymer obtained by polymerizing C1- to C4-alkyl esters of acrylic or of methacrylic acid and an alkyl ester of acrylic or of methacrylic acid with a tertiary or a quaternary ammonium group in the alkyl group; or
iv) a (meth)acrylate copolymer obtained by polymerizing methacrylic acid and ethyl acrylate, methacrylic acid and methyl methacrylate, ethyl acrylate and methyl methacrylate or methacrylic acid, methyl acrylate and methyl methacrylate; or
v) a (meth)acrylate copolymer obtained by polymerizing 40 to 60 % by weight of methacrylic acid and 60 to 40 % by weight of ethyl acrylate; or
vi) a (meth)acrylate copolymer obtained by polymerizing 60 to 80 % of ethyl acrylate and 40 to 20 % by weight of methyl methacrylate; or
vii) a (meth)acrylate copolymer obtained by polymerizing 5 to 15 % by weight methacrylic acid, 60 to 70 % by weight of methyl acrylate and 20 to 30 % by weight methyl methacrylate; or mixtures thereof.

5. Process according to claim 1 or 2, wherein the at least one polymer a1) and/or a2) is a mixture of
i) a (meth)acrylate copolymer obtained by copolymerizing 40 to 60 % by weight of methacrylic acid and 60 to 40 % by weight of ethyl acrylate and a (meth)acrylate copolymer obtained by polymerizing 60 to 80 % by weight of ethyl acrylate and 40 to 20 % by weight of methyl methacrylate and optionally up to 2 % by weight of (meth) acrylic acid; or
ii) a (meth)acrylate copolymer obtained by copolymerizing 5 to 15 % by weight methacrylic acid, 60 to 70 % by weight of methyl acrylate and 20 to 30 % by weight methyl methacrylate and a (meth)acrylate copolymer obtained by copolymerizing 40 to 60 % by weight of methacrylic acid and 60 to 40 % by weight of ethyl acrylate.

6. Process according to claim 1 or 2, wherein the at least one polymer a1) and/or a2) is selected from at least one anionic cellulose, ethyl cellulose or starch comprising at least 35 % by weight amylose or mixtures thereof.

7. Process according to claim 1 or 2, wherein the at least one polymer a1) and/or a2), preferably a2), is selected from celluloses, like hydroxyethyl cellulose (HEC), hydroxypropyl cellulose (HPC), hydroxypropyl methyl cellulose (HPMC), hydroxyethyl methyl cellulose (HEMC), ethyl cellulose (EC), methyl cellulose (MC), cellulose esters, cellulose glycolates, polyethylene glycols, polyethylene oxides, polyvinyl pyrrolidone, polyvinyl acetate, polyvinyl alcohol, or a mixture thereof.

8. Process according to any of the preceding claims, wherein at least one glidant is present in the first and/or second coating solution, suspension or dispersion.

9. Process according to any of the preceding claims, wherein at least one emulsifier is present in the first coating solution, suspension or dispersion.

10. Process according to any of the preceding claims, wherein at least one plasticizer is present in the first coating solution, suspension or dispersion.

11. Process according to any of the preceding claims, wherein up to 400 % by weight, based on the total weight of the at least one polymer, of at least one additive are comprised in the first and/or second coating solution, suspension or dispersion.

12. Process according to any of the preceding claims, wherein the body and the cap are comprising encircling notches or dimples in the area where the cap overlaps the body, that allow the capsule to be closed by a snap-into-place mechanism either in the pre-locked state or in the final-locked state.

13. Process according to any of the preceding claims, wherein the body comprises a tapered rim.

14. Polymer-coated hard shell capsule, obtained from a process according to any of claims 1 to 13.

15. Use of the polymer-coated hard shell capsule according to claim 14 for immediate, delayed or sustained release.

## Patentansprüche

1. Verfahren zur Herstellung einer polymerbeschichteten Hartschalenkapsel, die mindestens eine Funktionsbeschichtung und eine Deckbeschichtung umfasst und die als Behälter für pharmazeutische oder nutrazeutische biologische Wirkstoffe geeignet ist, wobei die Hartschalenkapsel einen Körper und eine Kappe umfasst, wobei die Kappe im verschlossenen Zustand den Körper entweder in einem vorverriegelten Zustand oder in einem endverriegelten Zustand überlappt, wobei die Hartschalenkapsel im vorverriegelten Zustand bereitgestellt wird und
mit einer ersten Beschichtungslösung, -Suspension oder - Dispersion beschichtet wird, die Folgendes umfasst oder daraus besteht
a1) mindestens ein Polymer;
b1) gegebenenfalls mindestens ein Gleitmittel;
c1) gegebenenfalls mindestens einen Emulgator;
d1) gegebenenfalls mindestens einen Weichmacher;
e1) gegebenenfalls mindestens einen biologischen Wirkstoff; und
f1) gegebenenfalls mindestens ein Additiv, das von a1) bis e1) verschieden ist;
um die Funktionsbeschichtung der Hartschalenkapsel im vorverriegelten Zustand zu erhalten; und danach mit einer zweiten Beschichtungslösung, -Suspension oder - Dispersion beschichtet wird, die von der ersten Beschichtungslösung, -Suspension oder -Dispersion verschieden ist, die Folgendes umfasst oder daraus besteht
a2) mindestens ein Polymer;
b2) gegebenenfalls mindestens ein Gleitmittel;
c2) mindestens einen Emulgator;
d2) mindestens einen Weichmacher;
e2) gegebenenfalls mindestens einen biologischen Wirkstoff; und
f2) gegebenenfalls mindestens ein Additiv, das von a2) bis e2) verschieden ist;
um die Deckschicht der Hartschalenkapsel im vorverriegelten Zustand zu erhalten, wobei
die gesamte Beschichtungsmenge 2,0 bis 10 mg/cm² beträgt; und
die Beschichtungsmenge der Deckschicht höchstens 40 % der Beschichtungsmenge der Funktionsschicht beträgt.

2. Verfahren nach Anspruch 1, wobei das Basismaterial des Körpers und der Kappe aus Hydroxypropylmethylcellulose, Stärke, Gelatine, Pullulan und einem Copolymer eines C1- bis C4-Alkylesters aus (Meth)Acrylsäure und (Meth)Acrylsäure ausgewählt ist.

3. Verfahren nach Anspruch 1 oder 2, wobei das mindestens eine Polymer a1) und/oder a2) aus mindestens einem (Meth)Acrylat-Copolymer ausgewählt ist.

4. Verfahren nach Anspruch 1 oder 2, wobei das mindestens eine Polymer a1) und/oder a2) Folgendes ist
i) ein Kern-Schale-Polymer, das ein Copolymer ist, das durch ein zweistufiges Emulsionspolymerisationsverfahren erhalten wird, mit einem Kern mit 70 bis 80 Gew.-%, der polymerisierte Einheiten aus 65 bis 75 Gew.-% Ethylacrylat und 25 bis 35 Gew.-% Methylmethacrylat umfasst, und mit einer Schale mit 20 bis 30 Gew.-%, die polymerisierte Einheiten aus 45 bis 55 Gew.-% Ethylacrylat und 45 bis 55 Gew.-% Methacrylsäure umfasst; oder
ii) ein anionisches Polymer, das erhalten wird, indem 25 bis 95 Gew.-% C1- bis C12-Alkylester von Acrylsäure oder Methacrylsäure und 75 bis 5 Gew.-% (Meth)AcrylatMonomere mit einer anionischen Gruppe polymerisiert werden; oder
iii) ein kationisches (Meth)Acrylat-Copolymer, das erhalten wird, indem C1- bis C4-Alkylester von Acryl- oder Methacrylsäure und ein Alkylester von Acryl- oder Methacrylsäure mit einer tertiären oder einer quaternären Ammonium-Gruppe in der Alkyl-Gruppe polymerisiert werden; oder
iv) ein (Meth)Acrylat-Copolymer, das erhalten wird, indem Methacrylsäure und Ethylacrylat, Methacrylsäure und Methylmethacrylat, Ethylacrylat und Methylmethacrylat oder Methacrylsäure, Methylacrylat und Methylmethacrylat polymerisiert werden; oder
v) ein (Meth)Acrylat-Copolymer, das erhalten wird, indem 40 bis 60 Gew.-% Methacrylsäure und 60 bis 40 Gew.-% Ethylacrylat polymerisiert werden; oder
vi) ein (Meth)Acrylat-Copolymer, das erhalten wird, indem 60 bis 80 Gew.-% Ethylacrylat und 40 bis 20 Gew.-% Methylmethacrylat polymerisiert werden; oder
vii) ein (Meth)Acrylat-Copolymer, das erhalten wird, indem 5 bis 15 Gew.-% Methacrylsäure, 60 bis 70 Gew.-% Methylacrylat und 20 bis 30 Gew.-% Methylmethacrylat polymerisiert werden; oder Mischungen davon.

5. Verfahren nach Anspruch 1 oder 2, wobei das mindestens eine Polymer a1) und/oder a2) eine Mischung ist aus
i) einem (Meth)Acrylat-Copolymer, das erhalten, wird, indem 40 bis 60 Gew.-% Methacrylsäure und 60 bis 40 Gew.-% Ethylacrylat copolymerisiert werden, und einem (Meth)Acrylat-Copolymer, das erhalten, wird, indem 60 bis 80 Gew.-% Ethylacrylat und 40 bis 20 Gew.-% Methylmethacrylat und gegebenenfalls bis zu 2 Gew.-% (Meth)Acrylsäure polymerisiert werden; oder
ii) einem (Meth)Acrylat-Copolymer, das erhalten wird, indem 5 bis 15 Gew.-% Methacrylsäure, 60 bis 70 Gew.-% Methylacrylat und 20 bis 30 Gew.-% Methylmethacrylat copolymerisiert werden, und einem (Meth)Acrylat-Copolymer, das erhalten wird, indem 40 bis 60 Gew.-% Methacrylsäure und 60 bis 40 Gew.-% Ethylacrylat copolymerisiert werden.

6. Verfahren nach Anspruch 1 oder 2, wobei das mindestens eine Polymer a1) und/oder a2) aus mindestens einer anionischen Cellulose, Ethylcellulose oder Stärke, die mindestens 35 Gew.-% Amylose umfasst, oder Mischungen davon ausgewählt ist.

7. Verfahren nach Anspruch 1 oder 2, wobei das mindestens eine Polymer a1) und/oder a2), vorzugsweise a2), ausgewählt ist aus Cellulosen, wie Hydroxyethylcellulose (HEC), Hydroxypropylcellulose (HPC), Hydroxypropylmethylcellulose (HPMC), Hydroxyethylmethylcellulose (HEMC), Ethylcellulose (EC), Methylcellulose (MC), Celluloseestern, Celluloseglycolaten, Polyethylenglykolen, Polyethylenoxiden, Polyvinylpyrrolidon, Polyvinylacetat, Polyvinylalkohol oder einer Mischung davon.

8. Verfahren nach einem der vorhergehenden Ansprüche, wobei in der ersten und/oder zweiten Beschichtungslösung, -Suspension oder -Dispersion mindestens ein Gleitmittel vorliegt.

9. Verfahren nach einem der vorhergehenden Ansprüche, wobei in der ersten Beschichtungslösung, -Suspension oder -Dispersion mindestens ein Emulgator vorliegt.

10. Verfahren nach einem der vorhergehenden Ansprüche, wobei in der ersten Beschichtungslösung, -Suspension oder -Dispersion mindestens ein Weichmacher vorliegt.

11. Verfahren nach einem der vorhergehenden Ansprüche, wobei in der ersten und/oder zweiten Beschichtungslösung, -Suspension oder -Dispersion bis zu 400 Gew.-%, bezogen auf das Gesamtgewicht des mindestens einen Polymers, mindestens eines Additivs vorliegen.

12. Verfahren nach einem der vorhergehenden Ansprüche, wobei der Körper und die Kappe umlaufende Kerben oder Vertiefungen in dem Bereich umfassen, in dem die Kappe den Körper überlappt, die ermöglichen, dass die Kapsel durch einen Einrastmechanismus entweder in den vorverriegelten Zustand oder in den endverriegelten Zustand verschlossen wird.

13. Verfahren nach einem der vorhergehenden Ansprüche, wobei der Körper einen sich verjüngenden Rand umfasst.

14. Polymerbeschichtete Hartschalenkapsel, die aus einem Verfahren nach einem der Ansprüche 1 bis 13 erhalten wird.

15. Verwendung der polymerbeschichteten Hartschalenkapsel nach Anspruch 14 zur sofortigen, verzögerten oder verlängerten Freisetzung.

## Revendications

1. Procédé de préparation d'une capsule à enveloppe dure enrobée de polymère comprenant au moins une couche fonctionnelle et une couche supérieure, convenant comme récipient pour des ingrédients biologiquement actifs pharmaceutiques ou nutraceutiques, dans lequel la capsule à enveloppe dure comprend un corps et un capuchon, dans lequel à l'état fermé, le capuchon chevauche le corps soit dans un état pré-verrouillé, soit dans un état final-verrouillé, dans lequel la capsule à enveloppe dure est fournie à l'état pré-verrouillé et est enrobée
par une première solution, suspension ou dispersion d'enrobage comprenant ou constituée de
a1) au moins un polymère ;
b1) éventuellement au moins un agent glissant ;
c1) éventuellement au moins un émulsifiant ;
d1) éventuellement au moins un plastifiant ;
e1) éventuellement au moins un ingrédient biologiquement actif ; et
f1) éventuellement au moins un additif, différent de a1) à e1) ;
pour obtenir l'enrobage fonctionnel de la capsule à enveloppe dure à l'état pré-verrouillé ; et ensuite est revêtue par une deuxième solution, suspension ou dispersion d'enrobage, qui est différente de la première solution, suspension ou dispersion d'enrobage, comprenant ou constituée de
a2) au moins un polymère ;
b2) éventuellement au moins un agent glissant ;
c2) au moins un émulsifiant ;
d2) au moins un plastifiant ;
e2) éventuellement au moins un ingrédient biologiquement actif ; et
f2) éventuellement au moins un additif, différent de a2) à e2) ;
pour obtenir la couche supérieure de la capsule à enveloppe dure à l'état pré-verrouillé, dans lequel
la quantité totale d'enrobage est de 2,0 à 10 mg/cm² ; et la quantité d'enrobage de la couche supérieure est d'au plus 40 % de la quantité d'enrobage de l'enrobage fonctionnel.

2. Procédé selon la revendication 1, dans lequel le matériau de base du corps et du capuchon est choisi parmi l'hydroxypropylméthylcellulose, l'amidon, la gélatine, le pullulane et un copolymère d'un ester d'alkyle en C1 à C4 d'acide (méth)acrylique et l'acide (méth)acrylique.

3. Procédé selon la revendication 1 ou 2, l'au moins un polymère a1) et/ou a2 étant choisi parmi au moins un copolymère de (méth)acrylate.

4. Procédé selon la revendication 1 ou 2 dans lequel l'au moins un polymère a1) et/ou a2) est
i) un polymère noyau-enveloppe, qui est un copolymère obtenu par un procédé de polymérisation en émulsion en deux stades avec un noyau avec 70 à 80 % en poids, comprenant des motifs polymérisés de 65 à 75 % en poids d'acrylate d'éthyle et de 25 à 35 % en poids de méthacrylate de méthyle, et une enveloppe avec 20 à 30 % en poids, comprenant des motifs polymérisés de 45 à 55 % en poids d'acrylate d'éthyle et de 45 à 55 % en poids d'acide méthacrylique ; ou
ii) un polymère anionique obtenu par polymérisation de 25 à 95 % en poids d'esters d'alkyle en C1- à C12 d'acide acrylique ou d'acide méthacrylique et de 75 à 5 % en poids de monomères de (méth)acrylate comportant un groupement anionique ; ou
iii) un copolymère de (méth)acrylate cationique obtenu par polymérisation d'esters d'alkyle en C1 à C4 d'acide acrylique ou méthacrylique et d'un ester d'alkyle d'acide acrylique ou d'acide méthacrylique comportant un groupe ammonium tertiaire ou un groupe ammonium quaternaire dans le groupe alkyle ; ou
iv) un copolymère de (méth)acrylate obtenu par polymérisation d'acide méthacrylique et d'acrylate d'éthyle, d'acide méthacrylique et de méthacrylate de méthyle, d'acrylate d'éthyle et de méthacrylate de méthyle ou d'acide méthacrylique, d'acrylate de méthyle et de méthacrylate de méthyle ; ou
v) un copolymère de (méth)acrylate obtenu par polymérisation de 40 à 60 % en poids d'acide méthacrylique et de 60 à 40 % en poids d'acrylate d'éthyle ; ou
vi) un copolymère de (méth)acrylate obtenu par polymérisation de 60 à 80 % d'acrylate d'éthyle et de 40 à 20 % en poids de méthacrylate de méthyle ; ou
vii) un copolymère de (méth)acrylate obtenu par polymérisation de 5 à 15 % en poids d'acide méthacrylique, de 60 à 70 % en poids d'acrylate de méthyle et de 20 à 30 % en poids de méthacrylate de méthyle ; ou des mélanges correspondants.

5. Procédé selon la revendication 1 ou 2 dans lequel l'au moins un polymère a1) et/ou a2) est un mélange de
i) un copolymère de (méth)acrylate obtenu par copolymérisation de 40 à 60 % en poids d'acide méthacrylique et de 60 à 40 % en poids d'acrylate d'éthyle et d'un copolymère de (méth)acrylate obtenu par polymérisation de 60 à 80 % en poids d'acrylate d'éthyle et de 40 à 20 % en poids de méthacrylate de méthyle et éventuellement jusqu'à 2 % en poids d'acide (méth)acrylique ; ou
ii) un copolymère de (méth)acrylate obtenu par copolymérisation de 5 à 15 % en poids d'acide méthacrylique, de 60 à 70 % en poids d'acrylate de méthyle et de 20 à 30 % en poids de méthacrylate de méthyle et d'un copolymère de (méth)acrylate obtenu par copolymérisation de 40 à 60 % en poids d'acide méthacrylique et de 60 à 40 % en poids d'acrylate d'éthyle.

6. Procédé selon la revendication 1 ou 2, dans lequel l'au moins un polymère a1) et/ou a2) est choisi parmi au moins une cellulose anionique, une éthylcellulose ou un amidon comprenant au moins 35 % en poids d'amylose ou des mélanges de ceux-ci.

7. Procédé selon la revendication 1 ou 2, dans lequel l'au moins un polymère a1) et/ou a2), préférablement a2), est choisi parmi des celluloses, comme l'hydroxyéthylcellulose (HEC), l'hydroxypropylcellulose (HPC), l'hydroxypropylméthylcellulose (HPMC), l'hydroxyéthylméthylcellulose (HEMC), l'éthylcellulose (EC), la méthylcellulose (MC), les esters de cellulose, les glycolates de cellulose, les polyéthylène glycols, les poly(oxydes d'éthylène), la polyvinylpyrrolidone, le poly(acétate de vinyle), le poly(alcool vinylique), ou un mélange de ceux-ci.

8. Procédé selon l'une quelconque des revendications précédentes, dans lequel au moins un agent glissant est présent dans la première et/ou la seconde solution, suspension ou dispersion d'enrobage.

9. Procédé selon l'une quelconque des revendications précédentes, dans lequel au moins un émulsifiant est présent dans la première solution, suspension ou dispersion d'enrobage.

10. Procédé selon l'une quelconque des revendications précédentes, dans lequel au moins un plastifiant est présent dans la première solution, suspension ou dispersion d'enrobage.

11. Procédé selon l'une quelconque des revendications précédentes, dans lequel jusqu'à 400 % en poids, par rapport au poids total de l'au moins un polymère, d'au moins un additif sont compris dans la première et/ou la seconde solution, suspension ou dispersion d'enrobage.

12. Procédé selon l'une quelconque des revendications précédentes, dans lequel le corps et le capuchon comprennent des encoches ou des creux d'encerclement dans la zone où le capuchon chevauche le corps, qui permettent à la capsule d'être fermée par un mécanisme d'encliquetage soit dans l'état pré-verrouillé, soit dans l'état final-verrouillé.

13. Procédé selon l'une quelconque des revendications précédentes, dans lequel le corps comprend un bord effilé.

14. Capsule à enveloppe dure enrobée de polymère, obtenue par un procédé selon l'une quelconque des revendications 1 à 13.

15. Utilisation de la capsule à enveloppe dure enrobée de polymère selon la revendication 14 pour une libération immédiate, retardée ou prolongée.
